# EUROPEAN PATENT SPECIFICATION

(11) **EP 1 109 805 B1**
(45) Date of publication and mention of the grant of the patent: **17.12.2003**
(21) Application number: 99933622.5
(22) Date of filing: 30.06.1999
(51) Int. Cl.: C07D 401/12, A61K 31/40, A01N 43/38, C07D 401/06, C07D 209/08, C07D 209/12, C07D 209/42, C07D 213/80, C07C 229/42, C07C 235/64

(54) **INHIBITORS OF BACTERIAL NAD SYNTHETASE**
INHIBITOREN DER BAKTERIELLEN NAD SYNTHETASE
INHIBITEURS DE LA NAD SYNTHETASE BACTERIENNE

(30) Priority: 25.08.1998 US 97880 P; 14.01.1999 WO PCT/US99/00810
(43) Date of publication of application: 27.06.2001
(73) Proprietor: THE UAB RESEARCH FOUNDATION, Birmingham, AL 35294-0111 (US)
(72) Inventor: BROUILLETTE, Wayne, J., Pelham, AL 35124 (US); MUCCIO, Donald, Hoover, AL 35226 (US); JEDRZEJAS, Mark, J., Birmingham, AL 35124 (US); BROUILLETTE, Christie, G., Pelham, AL 35124 (US); DEVEDJIEV, Yancho, Charlottesville, VA 22901 (US); CRISTOFOLI, Walter, Birmingham, AL 35205 (US); DELUCAS, Lawrence, J., Birmingham, AL 35243 (US); GARCIA, Jose Gabriel, Birmingham, AL 35205 (US); SCHMITT, Laurent, Lafayette, IN 47905 (US); VELU, Sadanandan, E., Birmingham, AL 35209 (US)
(74) Representative: Gates, Marie Christina Esther
(86) International application number: US9914839
(87) International publication number: WO00010996

(56) References cited:
- EP-A- 0 585 722
- US-A- 4 289 777

## Description

The present invention pertains to antibacterial and antimicrobial agents. In particular, the present invention provides methods of synthesizing and screening compounds that are bacterial nicotinamide adenine dinucleotide (NAD) synthetase enzyme inhibitors. The present invention also provides novel compounds that inhibit bacterial NAD synthetase enzyme. The invention also provides libraries of compounds that comprise bacterial NAD synthetase enzyme inhibitors. Further, the present invention provides compounds that exhibit therapeutic activity as antibacterial agents, antimicrobial agents and broad spectrum antibiotics. Still further, the invention provides methods of treating a mammal with bacterial NAD synthetase enzyme inhibitor compounds. The present invention also provides novel disinfecting agents.

### BACKGROUND OF THE INVENTION

Drug-resistant infectious bacteria, that is, bacteria that are not killed or inhibited by existing antibacterial and antimicrobial compounds, have become an alarmingly serious worldwide health problem. (E. Ed. Rubenstein, *Science*, **264**, 360 (1994)). In fact, a number of bacterial infections may soon be untreatable unless alternative drug treatments are identified.

Antimicrobial or antibacterial resistance has been recognized since the introduction of penicillin nearly 50 years ago. At that time, penicillin-resistant infections caused by *Staphylococcus aureus* rapidly appeared. Today, hospitals worldwide are facing unprecedented crises from the rapid emergence and dissemination of microbes resistant to one or more antimicrobial and antibacterial agents commonly in use today. As stated in the Fact Sheet on Antimicrobial Resistance of the National Institute of Allergy and Infectious Diseases, National Institutes of Health, several strains of antibiotic-resistant bacteria are now emerging and are becoming a threat to human and animal populations, including those summarized below:
1) Strains of *Staphylococcus aureus* resistant to methicillin and other antibiotics are endemic in hospitals. Infection with methicillin-resistant *S. aureus* (MRSA) strains may also be increasing in non-hospital settings. Vancomycin is the only effective treatment for MRSA infections. A particularly troubling observation is that *S. aureus* strains with reduced susceptibility to vancomycin have emerged recently in Japan and the United States. The emergence of vancomycin-resistant strains would present a serious problem for physicians and patients.
2) Increasing reliance on vancomycin has led to the emergence of vancomycin-resistant *enterococci* (VRE), bacteria that infect wounds, the urinary tract and other sites. Until 1989, such resistance had not been reported in U.S. hospitals. By 1993, however, more than 10 percent of hospital-acquired *enterococci* infections reported to the Centers for Disease Control ("CDC") were resistant.
3) *Streptococcus pneumoniae* causes thousands of cases of meningitis and pneumonia, as well as 7 million cases of ear infection in the United States each year. Currently, about 30 percent of *S. pneumoniae* isolates are resistant to penicillin, the primary drug used to treat this infection. Many penicillin-resistant strains are also resistant to other antimicrobial or antibacterial drugs.
4) Strains of multi-drug resistant tuberculosis (MDR-TB) have emerged over the last decade and pose a particular threat to people infected with HIV. Drug-resistant strains are as contagious as those that are susceptible to drugs. MDR-TB is more difficult and vastly more expensive to treat, and patients may remain infectious longer due to inadequate treatment. Multi-drug resistant strains of *Mycobacterium tuberculosis* have also emerged in several countries, including the U.S.
5) Diarrheal diseases cause almost 3 million deaths a year, mostly in developing countries, where resistant strains of highly pathogenic bacteria such as *Shigella dysenteriae, Campylobacter, Vibrio cholerae, Escherichia coli* and *Salmonella* are emerging. Furthermore, recent outbreaks of *Salmonella* food poisoning have occurred in the United'States. A potentially dangerous "superbug" known as *Salmonella typhimurium*, resistant to ampicillin, sulfa, streptomycin, tetracycline and chloramphenicol, has caused illness in Europe, Canada and the United States.

In addition to its adverse effect on public health, antimicrobial or antibacterial resistance contributes to higher health care costs. Treating resistant infections often requires the use of more expensive or more toxic drugs and can result in longer hospital stays for infected patients. The Institute of Medicine, a part of the National Academy of Sciences, has estimated that the annual cost of treating antibiotic resistant infections in the United States may be as high as $30 billion.

Given the above, it would be highly desirable to develop novel antibacterial and antimicrobial agents that act by different mechanisms than those agents in use currently. Further, it would be desirable to be able to synthesize such novel compounds. It would also be desirable to develop libraries of compounds that exhibit inhibitory bacterial NAD synthetase activity. Such new agents would be useful to counteract antibiotic resistant strains of bacteria and other types of harmful microbes. It would be even more desirable to develop antibacterial agents that inhibit or block essential bacterial metabolic mechanisms, to result in bacterial death or deactivation, without also affecting the essential metabolic activities of a mammalian host. That is, it would be desirable to develop antibacterial agents that preferentially attack bacteria and other microbes and kill or deactivate the harmful organism without causing any attendant undesirable side effects in a human or animal patient. It would also be desirable to develop methods of rapidly screening potential new antimicrobial and antibacterial agents. It would also be desirable to develop novel disinfecting agents.

EP-A-585722 describes anti-microbial agents having the following chemical structure in which n is 0 or 1: -

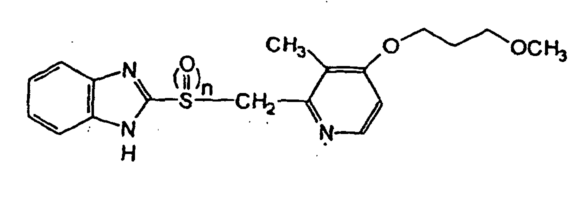

### SUMMARY OF THE INVENTION

In one aspect the invention provides A compound of the formula: 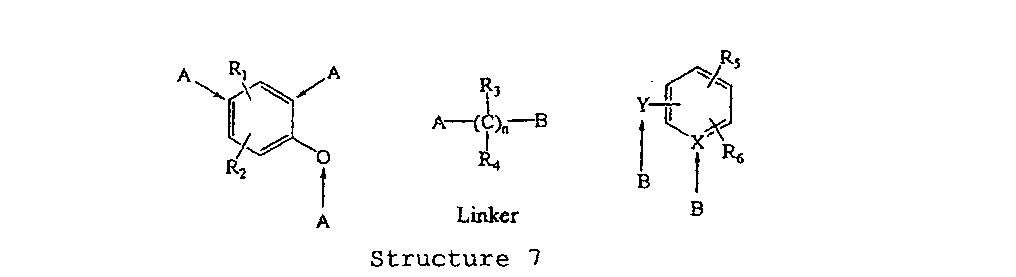 wherein X is C, N, O, or S, Y is C, N, O, S, carboxy, ester, amide, or ketone, A and B represent the respective sites of attachment for a linker,
n is an integer of from 1 to 12, and
R₁-R₆ each, independently, is H,
aryl, hydroxy, ketone, nitro, amino, amidino, guanidino, carboxylate, amide, sulfonate, halogen, a substituted or unsubstituted branched, un-branched or cyclic aliphatic group,
the linker is a branched, un-branched or cyclic aliphatic group, wherein aliphatic groups include alkyl, alkenyl and alkynyl groups,
the linker can further include heteroatoms, when R₁-R₆ represents a cyclic group, the group can include one or more closed rings, and
the compound inhibits the NAD synthetase enzyme of a microbe.

n may be an integer from 3 to 10, preferably from 5 to 9, more preferably from
6 to 9. The groups R1 - R6 may each independantly be H, alkyl, alkenyl, alkynyl, aryl, hydroxyl, ketone, nitro, amino, amidino, guanidino, carboxylate, amide, sylfonate or halogen or a common derivative of these groups.

In particular the inventions provides compounds in which X⁻ is an anion of the formulae 2;- 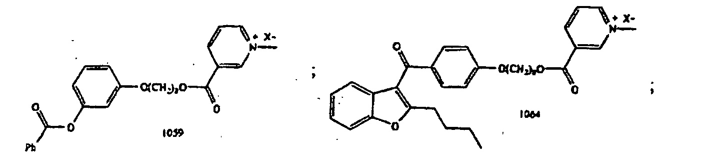 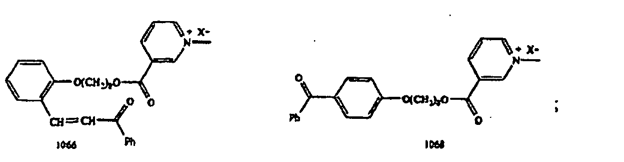 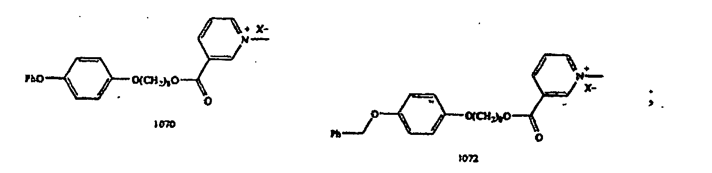 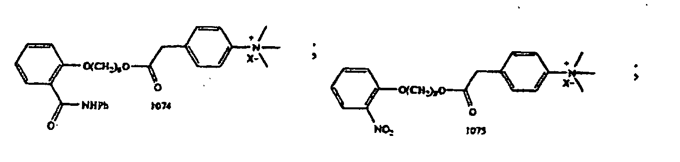 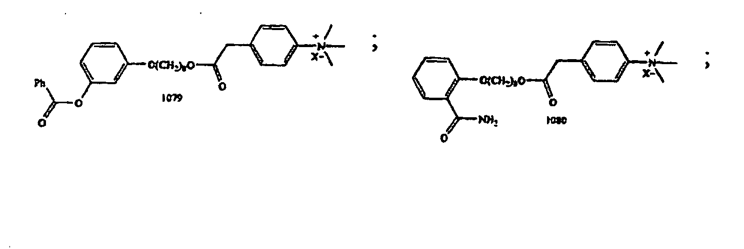 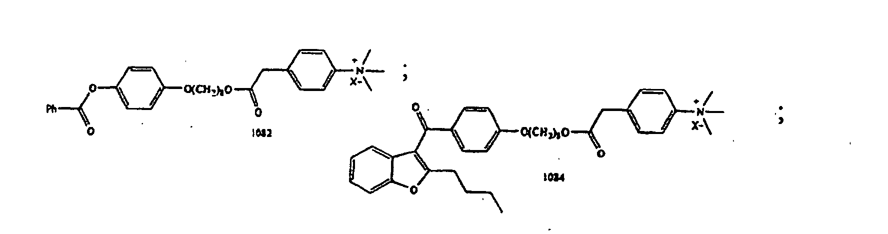 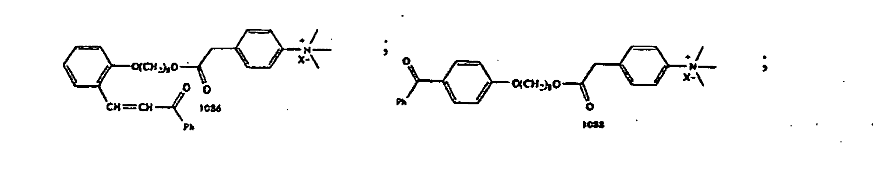 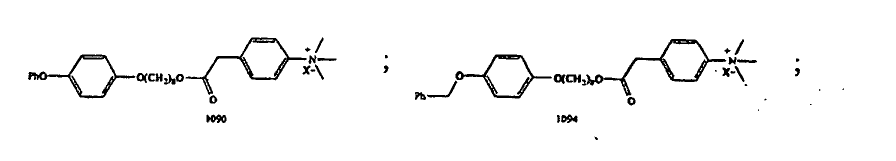 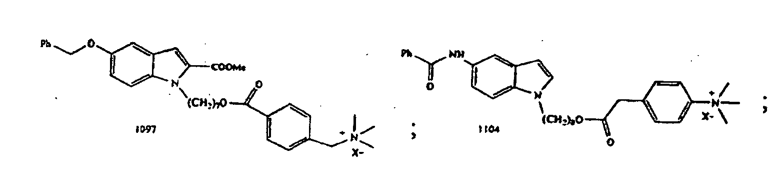 or

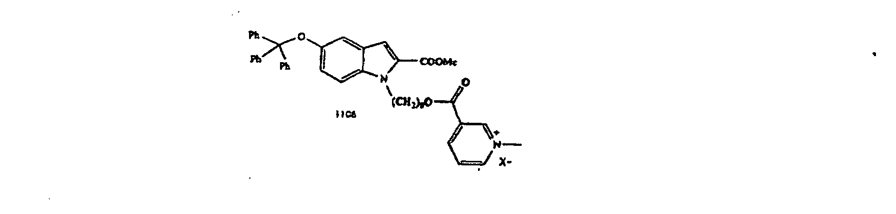

Further, the invention provides the use of the above compounds in the manufacture of a medicament for treating or preventing a microbial infection in a mammal, particularly a prokaryote is infection. Moreover, a method is provided of decreasing prokaryotic growth, comprising contacting the prokaryote with an amount of a prokaryotic NAD synthetase enzyme inhibitor effective to reduce or eliminate the production of NAD whereby prokaryotic growth is decreased. Further provided is a disinfectant compound wherein the compound comprises a bacterial NAD synthetase enzyme inhibitor. Still further, the invention provides a method of disinfecting a material contaminated by a microbe, comprising contacting a contaminated material with a bacterial NAD synthetase enzyme inhibitor compound in an amount sufficient to kill or deactivate the microbe.

Still further, the invention provides a method of making a bacterial NAD synthetase inhibitor compound comprising the steps of: a. brominating an aniline with N-bromosuccinimide to form a 2-bromo-R¹-substituted-aniline or a 2-bromo-R²-substituted-aniline; b. reacting the 2-bromo-R¹-substituted-aniline or a 2-bromo-R²-substituted-aniline using a Heck coupling reaction to form an alkyne-substituted aniline; c. reacting the alkyne-substituted aniline using a cyclization reaction to form an indole alcohol; d. quaternizing the indole alcohol with an amine; e.reacting the indole alcohol with triflouromethylsulfonic anhydride to provide a triflate; and f. reacting the indole triflate with an amine to form an indole alkylammonium product.

In a further aspect, the invention provides a method of making a bacterial NAD synthetase inhibitor compound comprising the steps of: a. alkylating a phenol with 7-bromo-1-heptanol to provide 7-(phenyloxy)-1-heptanol; b. mesylating 7-(phenyloxy)-1-heptanol to provide 7-(phenyloxy)-1-heptyl methanesulfonate; c. esterifying 7-(phenyloxy)-1-heptyl-methanesulfonate to provide 7-(phenyloxy)-1-heptyl nicotinate; and d. n-methylating 7-(phenyloxy)-1-heptyl nicotinate to provide [7-(phenyloxy)-1-heptyl-(N-methyl) nictotinate] iodide.

Mentioned in the following but not claimed is a method of generating a library comprising at least one bacterial NAD synthetase enzyme inhibitor compound comprising the steps of: a. obtaining the crystal structure of a bacterial NAD synthetase enzyme; b. identifying one or more sites of catalytic activity on the NAD synthetase enzyme; c. identifying the chemical structure of the catalytic sites on the NAD synthetase enzyme; d. selecting one or more active molecules that will demonstrate affinity for at least one of the catalytic sites on the NAD synthetase enzyme; f. synthesizing one or more dimeric compounds comprised of at least one active molecule wherein the active molecule compound are joined by means of n linker compounds and wherein n is an integer of from 1 to 12, and g. screening the one or more compounds for NAD synthetase inhibitor activity.

In a further aspect a method is provided for the in vitro screening a compound for bacterial NAD synthetase enzyme inhibitory activity comprising the steps of: a. preparing a bacterial NAD synthetase enzyme solution from pure bacterial NAD synthetase enzyme mixed with a suitable buffer; b. contacting the bacterial NAD synthetase enzyme solution with a test compound; and c. measuring the rate of the enzyme-catalyzed reaction between the NAD synthetase enzyme and the test compound, wherein the rate of the enzyme catalyzed reaction comprises a measure of bacterial NAD synthetase enzyme inhibitory activity.

Additional advantages of the invention will be set forth in part in the description that follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION OF THE INVENTION

The present invention may be understood more readily by reference to the following detailed description of preferred embodiments of the invention and the Examples included herein.

Before the present methods, compounds, compositions and apparatuses are disclosed and described it is to be understood that this invention is not limited to the specific synthetic methods described herein. It is to be further understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment.

Throughout this application, where a chemical diagram has a straight line emanating from a chemical structure, such a line represents a CH₃ group. For example, in the following diagram: 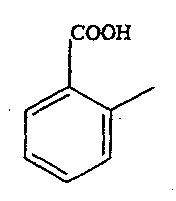 *o*-methylbenzoic acid is represented.

The term "alkyl" as used herein refers to a branched or unbranched saturated hydrocarbon group of 1 to 24 carbon atoms, such as methyl, ethyl, *n*-propyl, isopropyl, *n*-butyl, isobutyl, *t*-butyl, octyl, decyl, tetradecyl, hexadecyl, eicosyl, tetracosyl and the like. The term "cycloalkyl" intends a cyclic alkyl group of from three to eight, preferably five or six carbon atoms.

The term "alkoxy" as used herein intends an alkyl group bound through a single, terminal ether linkage; that is, an "alkoxy" group may be defined as -OR where R is alkyl as defined above. A "lower alkoxy" group intends an alkoxy group containing from one to six, more preferably from one to four, carbon atoms.

The term "alkylene" as used herein refers to a difunctional saturated branched or unbranched hydrocarbon chain containing from 1 to 24 carbon atoms, and includes, for example, methylene (-CH2-), ethylene (-CH2-CH2-), propylene (-CH2-CH2-CH2-), 2-methylpropylene [-CH2-CH(CH3)-CH2-], hexylene [-(CH2)6-] and the like. The term "cycloalkylene" as used herein refers to a cyclic alkylene group, typically a 5- or 6-membered ring.

The term "alkene" as used herein intends a mono-unsaturated or di-unsaturated hydrocarbon group of 2 to 24 carbon atoms. Asymmetric structures such as (AB)C=C(CD) are intended to include both the E and Z isomers. This may be presumed in structural formulae herein wherein an asymmetric alkene is present.

The term "alkynyl" as used herein refers to a branched or unbranched unsaturated hydrocarbon group of 1 to 24 carbon atoms wherein the group has at least one triple bond.

The term "cyclic" as used herein intends a structure that is characterized by one or more closed rings. As further used herein, the cyclic compounds discussed herein may be saturated or unsaturated and may be heterocyclic. By heterocyclic, it is meant a closed-ring structure, preferably of 5 or 6 members, in which one or more atoms in the ring is an element other than carbon, for example, sulfur, nitrogen, etc.

The term "bicyclic" as used herein intends a structure with two closed rings. As further used herein, the two rings in a bicyclic structure can be the same or different. Either of the rings in a bicyclic structure may be heterocyclic.

By the term "effective amount" of a compound as provided herein is meant a sufficient amount of the compound to provide the desired treatment or preventive effect. As will be pointed out below, the exact amount required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount may be determined by one of ordinary skill in the art using only routine experimentation. It is preferred that the effective amount be essentially nontoxic to the subject, but it is contemplated that some toxicity will be acceptable in some circumstances where higher dosages are required.

By "pharmaceutically acceptable carrier" is meant a material that is not biologically or otherwise undesirable, i.e., the material may be administered to an individual along with the compounds of the invention without causing any undesirable biological effects or interacting in a deleterious manner with any of the other components of the pharmaceutical composition in which it is contained.

As used herein, "NAD synthetase enzyme" is defined as the enzyme that catalyzes the final reaction in the biosynthesis of NAD, namely, the transformation of NaAD into NAD. As used herein, the term "catalytic sites" are defined as those portions of the NAD synthetase enzyme that bind to substrates, and cofactors, including nictonic acid dinucleotide (NaAD), NAD, adenosine triphosphate (ATP), adenosine monophosphate (AMP), pyrophosphate, magnesium and ammonia in bacteria or microbes. The term "receptor site" or "receptor subsite" relates to those portions of the bacterial NAD synthetase enzyme in which the bacterial NAD synthetase enzyme inhibitors disclosed herein are believed to bind. For the purposes of this disclosure, the terms "catalytic site," "receptor site" and "receptor subsite" may be used interchangeably.

As used herein, the terms "library" and "library of compounds" denote an intentionally created collection of differing compounds which can be prepared by the synthetic means provided herein or generated otherwise using synthetic methods utilized in the art. The library can be screened for biological activity in any variety of methods, such as those disclosed below herein, as well as other methods useful for assessing the biological activity of chemical compounds. One of skill in the art will recognize that the means utilized to generate the libraries herein comprise generally combinatorial chemical methods such as those described in *Gallop*, *et al*, "Applications of Combinatorial Techniques to Drug Discovery," "Part 1 Background and Peptide Combinatorial Libraries," and "Part 2: Combinatorial Organic Synthesis, Library Screening Strategies, and Future Directions," *J. Med. Chem*., Vol. 37(1994) pp. 1233 and 1385. As used herein, the terms "combinatorial chemistry" or "combinatorial methods" are defined as the systematic and repetitive, covalent connection of a set of different "building blocks" of varying structure, such as the active molecules disclosed herein, to provide a large array of diverse molecular entities. As contemplated herein, the large array of diverse molecular entities together form the libraries of compounds of the invention.

As used herein, the term "antibacterial compound" denotes a material that kills or deactivates bacteria or microbes so as to reduce or eliminate the harmful effects of the bacteria on a subject or in a system. Such materials are also known in the art as 'bacteriostatic agents" or "bateriocidal agents." The bacteria so affected can be gram positive, gram negative or a combination thereof. The terms "antimicrobial compound" and "broad spectrum antibiotic" denote a material that kills or deactivates a wide variety of microbes, including, but not limited to, one of more of, gram positive or gram negative bacteria, *Staphylococcus aureus, Streptococcus pyogenes, Streptococcus viridans, Enterococcus, anaerobic Streptococcus, Pneumococcus, Gonococcus, Meningococcus, Mima, Bacillus anthracis, C. diphtheriae, List. monocytogenes, Streptobacillus monohiliformis, Erysipelothrix insidiosa, E. coli, A. aerogenes. A. faecalis, Proteus mirabilis, Pseudomonas aeruginosa, K. pneumoniae, Salmonella, Shigella, H. influenzae,* *H. ducreyi, Brucella. Past. pestis, Past. tularensis, Past. multocida, V. comma, Actinobacillus mallei, Pseud. pseudomallei, Cl. tetani, Bacteroides, Fusobacterium fusiforme. M. tuberculosis, atypical mycobacteria, Actinomyces israelii, Nocardia, T*. *pallidum, T. pernue, Borrelia recurrentis, Peptospira, Rickettsia, and Mycoplasma pneumoniae*.

In accordance with the desirability for developing improved antibacterial and antimicrobial agents, with the invention herein novel compounds have been identified that inhibit bacterial NAD synthetase enzymatic activity. Such activity translates into effectiveness as bacteriocidal agents, as well as effectiveness as a broad spectrum antibiotic materials. Novel compounds have been developed that inhibit a previously unrecognized target in prokaryotic organisms, such as bacteria, to block essential biological function and thereby cause bacterial death or deactivation of bacteria or other microbes. Specifically, the invention herein has identified an enzyme found in both gram positive and gram negative bacteria, NAD synthetase enzyme, which can be utilized as a target for drug design to provide protection from and/or treatment for bacterial and other microbial infections.

The NAD synthetase enzyme catalyzes the final step in the biosynthesis of nicotinamide adenine dinucleotide (NAD). Bacterial NAD synthetase is an ammonia-dependent amidotransferase belonging to a family of "N-type" ATP pyrophosphatases; this family also includes asparagine synthetase and argininosuccinate synthetase. NAD synthetase enzyme catalyzes the last step in both the *de novo* and salvage pathways for NAD⁺ biosynthesis, which involves the transfer of ammonia to the carboxylate of nicotinic acid adenine dinucleotide (NaAD) in the presence of ATP and Mg⁺². The overall reaction is illustrated in Scheme 1.

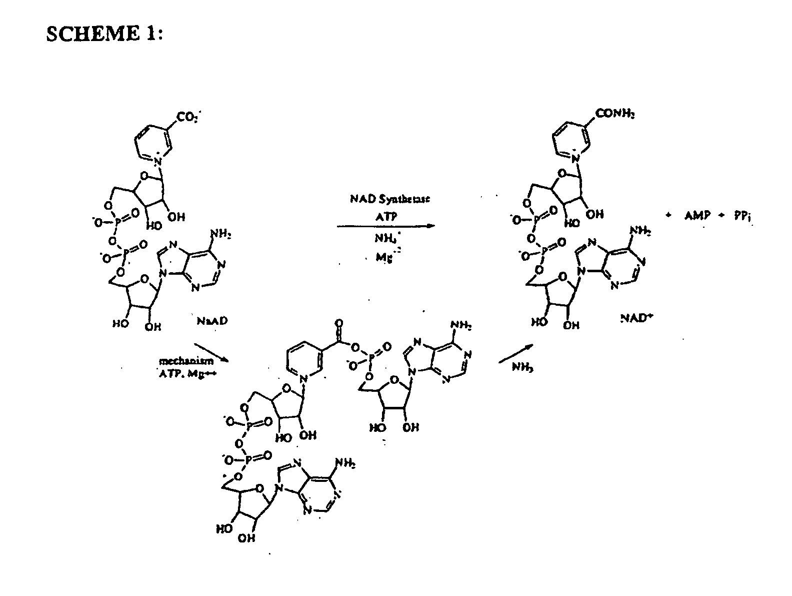

Unlike eukaryotic NAD synthetase *e*.*g*., that found in mammals, which can utilize glutamine as a source of nitrogen, prokaryotic NAD synthetase in bacteria utilizes ammonia as the sole nitrogen source. Through x-ray crystallography and other methods, the invention has identified marked differences in the structures of eukaryotic and prokaryotic forms of the NAD synthetase enzyme. For example, *B. subtilis* NAD synthetase enzyme, which in the invention has been crystallized and used in the drug design methodologies herein, is a dimeric material with molecular weight around 60,500. In marked contrast, the eukaryotic form of NAD synthetase found in mammals is multimeric and has a molecular weight of at least 10 times larger.

By utilizing the significant differences between the eukaryotic and prokaryotic forms of NAD synthetase enzyme, the invention herein provides novel compounds that can be utilized as antibacterial and antimicrobial agents that specifically target the prokaryotic NAD synthetase enzyme without also effecting a mammalian host. With the invention herein, it has been found that by specifically inhibiting bacterial NAD synthetase enzymatic activity, bacteria can be deprived of the energy necessary to thrive and replicate. Accordingly, through the invention disclosed and claimed herein, antibacterial and antimicrobial drugs have been developed that preferentially attack the bacteria to kill or deactivate it so as to reduce or eliminate its harmful properties, without appreciably affecting mammalian NAD synthetase enzymatic activity at the same dosage. Furthermore, novel methods are provided that allow the rapid screening of compounds for bacterial NAD synthetase enzyme inhibitory activity. Moreover, the invention provides methods of treating microbial infections in a subject. Because of the differences in structure between bacterial and mammalian NAD synthetase enzyme, it would not be expected that the compounds of the invention would inhibit or otherwise affect mammalian NAD synthetase enzyme in the same manner as the compounds act on bacteria.

Without being bound by theory, through chemical analysis and x-ray crystallography methods, at least two separate catalytic subsites on the bacterial NAD synthetase enzyme in which it is possible to bind at least one or more small molecules ("active molecules") have been characterized. These sites are illustrated below by the cartoon in Figure 2.

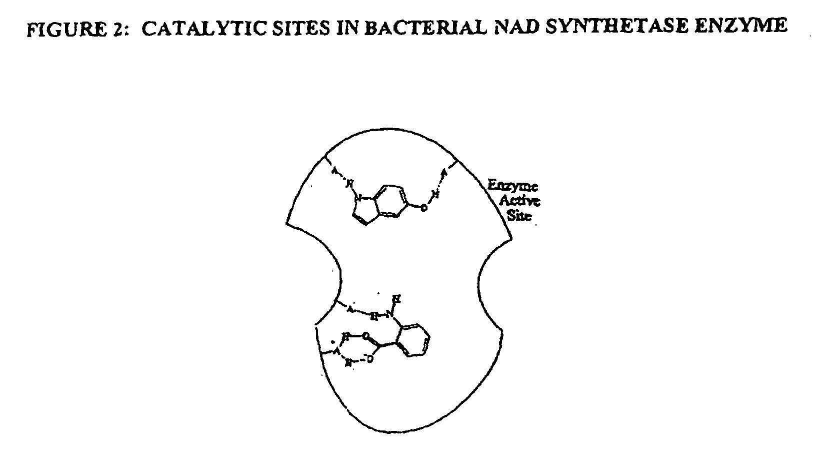

Because of the specific structure of these catalytic sites, it has been determined that it is possible to identify small molecules that will demonstrate affinity for at least one of the sites. Small molecules of the proper configuration, the configuration being determined by the structure of the catalytic site(s), will bind with a receptor site or sites on the bacterial NAD synthetase enzyme, thereby blocking the catalytic activity of the enzyme. Figure 4 illustrates *via* cartoon a bacterial NAD synthetase enzyme in which the catalytic sites are blocked by an example of a compound of the present invention.

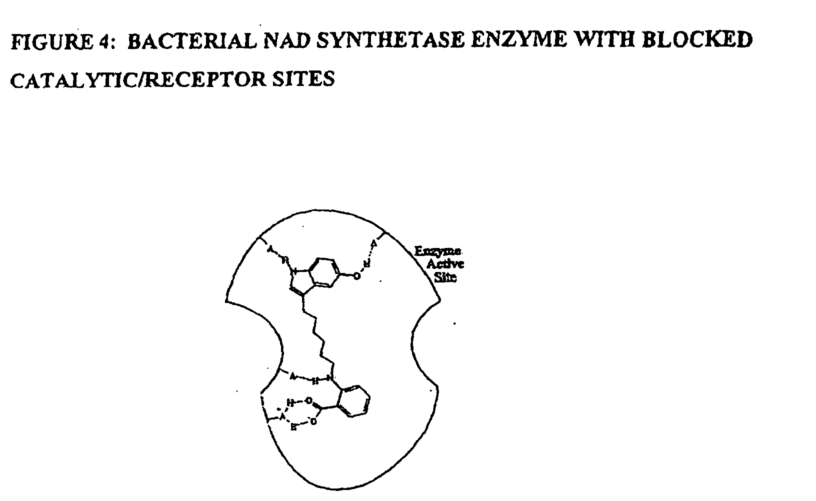

Under such circumstances, it is hypothesized that spore-forming bacteria will be unable to undergo germination and outgrowth, and the essential cellular respiratory functions of the vegetative bacteria will be halted, thereby causing cellular death or deactivation, *e*.*g*., gram positive and gram negative bacteria and other microbes will be killed or prevented from undergoing growth. Accordingly, the invention has found that compounds that exhibit inhibitory activity against the bacterial NAD synthetase enzyme will also exhibit therapeutic activity as antibacterial and antimicrobial compounds, as well as broad spectrum antibiotic materials.

With the invention herein it has been surprisingly found that it is possible to synthesize novel tethered dimeric compounds that will exhibit activity as bacterial NAD synthetase enzyme inhibitors. By linking one or more active molecules through a linker molecule, one or more ends of the tethered dimer can bind in the respective receptor sites or subsites to thereby render the bacterial NAD synthetase enzyme inactive. When more than one active molecule is used, each active molecule can be the same or different. The term "active molecules" as used herein refers to small molecules that may be used alone or tethered together through a linker (tether) fragment to form a tethered dimeric compound.

In the present invention, the active molecules are comprised of substituent groups as hereinafter disclosed that will bind with at least one of the receptor sites in bacterial NAD synthetase enzyme. In the invention herein one or more active molecules are tethered together to form a dimeric molecule that is capable of inhibiting the bacterial NAD synthetase enzyme.

Further, in this invention it has been found that, under some circumstances, different active molecules will be more likely to bind to different locations in the receptor site of a bacterial NAD synthetase enzyme because of the differing chemical make-up of each of these sites. Therefore, in one embodiment, it is beneficial to tether at least two different active molecules to each other wherein each active molecule demonstrates selective affinity for a different subsite in the receptor. Using the tethered dimers herein it is possible to drastically enhance the potency ofNAD synthetase enzyme inhibition, as compared to blocking a single site on the bacterial NAD synthetase enzyme. As used herein, the term "selective affinity" means that the active molecule shows enhanced tendency to bind with one subsite with the receptor in the bacterial NAD synthetase enzyme because of a chemical complementarity between the receptor subsite and the active molecule. A tethered dimer compound is illustrated in Scheme 2 below.

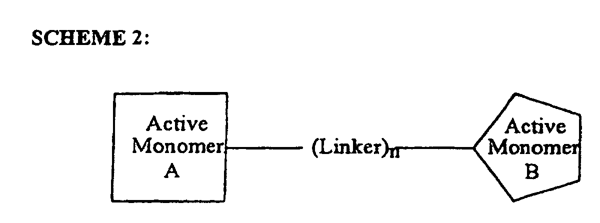

In one embodiment, a dimeric inhibitor compound will bind with, for example, the sites of catalytic activity on the bacterial NAD synthetase enzyme, thereby preventing the production of NAD/NADH by the bacteria. As an additional surprising finding in this invention, it has been determined that by varying the length of the linker molecule, and, accordingly, the distance between the two active molecules, the affinity of the tethered inhibitor compound for the NAD synthetase enzyme will also vary.

In practice of the invention relating to the design of novel NAD synthetase enzyme inhibitor compounds, a software program can be utilized which facilitates the prediction of the binding affinities of molecules to proteins so as to allow identification of commercially available small molecules with the ability to bind to at least one receptor subsite in the bacterial NAD synthetase enzyme. An example of one such computer program is DOCK, available from the Department of Pharmaceutical Chemistry at the University of California, San Francisco. DOCK evaluates the chemical and geometric complementarity between a small molecule and a macromolecular binding site. However, such a program would be useless in the design of a bacterial NAD synthetase enzyme inhibitor in the absence of complete information regarding the enzyme's structure and the chemical makeup of the receptor sites, identified and disclosed fully for the first time herein.

With this invention, the crystal structure of one type of bacterial NAD synthetase enzyme *e*.*g*., *B. subtilis* has been for the first time identified fully. The x-ray crystal structure of NAD synthetase enzyme from *B. subtilis* had been reported in the literature. (M. Rizzi *et al*., **The EMBO Journal,** 15, 5125, (1996); M. Rizzi *et al*., **Structure,** 1129 (1998)). This was accomplished in free form and in complex with ATP and Mg⁺² at 2.6 and 2.0 Å, respectively. This structure contained the hydrolyzed form of ATP, namely AMP and Ppi, in the ATP binding site and ATP was present in the NaAD binding site. However, the prior art was not able to obtain the structure of the enzyme complex containing NaAD due to technical problems that precluded full identification. Without the structure of the enzyme complex containing NaAD, the structure-based drug design targeted to NAD synthetase enzyme of the present invention could not be developed.

In order to carry out structure-based drug design targeted to bacterial NAD synthetase enzyme, the structure of the enzyme in complex with all substrates, including NaAD has been solved herein. The additional structural information obtained in this invention for the first time clearly defined the interactions between NaAD and the enzyme, which provided information important for guiding combinatorial library design and inhibitor identification. Schematic drawings of crystal structures of the open and blocked receptor/catalytic sites of *B. subtilis* are set out previously in Figures 2 and 4.

The invention utilizes two approaches reported in the literature (for other biological targets) to help identify lead compounds. (1) Once the structure of a bacterial NAD synthetase catalytic site was identified, the software DOCK (I.D. Kunz *et al*., **J. Mol. Biol.**, 161, 269-288 (1982)) was utilized to search the Available Chemicals Directory database and computationally score the relative binding affinities for each structure. Based on these results and structural information regarding substrate binding, commercially available compounds were selected for purchase and subsequent enzyme kinetics evaluation. Such database searching strategies in drug discovery are now commonly used by those of skill in the art of drug design. (D.T. Manallack, **Drug Discovery Today**, 1, 231-238 (1996)). (2) Using the results of biological screening for selected commercially available compounds to identify biologically active molecules, the inventors then designed a combinatorial library consisting of "tethered dimers" to rapidly identify more effective inhibitors of NAD synthetase enzyme as antibacterial agents. The use of "tethered dimers" to enhance the binding affinity of two moderately effective small molecule ligands that interact in the same binding site has been previously described in the literature. (S.B. Stuker, P.J. Hejduk, R.P. Meadows, and S.W. Fesik, **Science**, 274, 1531-1534 (1996)). However, this invention involves the first and, therefore, a novel application of database searching coupled with a combinatorial tethered dimer approach that was guided by the structure of and targeted to the bacterial NAD synthetase enzyme.

Examples from the top scoring small molecules as determined by, for example, DOCK, are preferably pre-screened using *in vitro* enzyme assays as further described herein. As a significant aspect of the invention herein, the preferred screening method utilized should allow the rapid screening of large numbers of compounds for inhibitory activity. In a preferred method of the present invention, the small molecule inhibitor candidate for each site that is most promising as an active molecule, as identified by DOCK (or other programs known to one of skill in the art) and the prescreening method herein, or that were designed based upon the substrate protein complex structure, were synthesized according to the methods disclosed herein below.

In one embodiment, the active molecules are chemically tethered to one another by means of a linker compound. In a further embodiment, the linker comprises one,or more CH₂ or other groups, using a variety of tether lengths, preferably 1 to 12 nonhydrogen atoms, more preferably 3 to 10 nonhydrogen atoms, further more preferably 5 to 9 nonhydrogen atoms and, still more preferably, 6 to 9 nonhydrogen atoms.

In another embodiment of the present invention, the novel compounds with preferred structures determined from the methods described above are synthesized by means of rapid, solution phase parallel synthesis of the tethered dimers compounds in a combinatorial fashion. One of skill in the art will recognize such techniques. For each class of dimeric compounds designed in accordance with the invention herein, a novel synthetic strategy was developed to allow variation in the length of the linking group through which the active molecules are joined. These synthetic strategies are set forth herein as Schemes 3 through 7 and in Examples 1 through 5 below. Use of the preferred method of variable linkage greatly increases the number of different tethered dimeric compounds that can be produced from a single pair of the same or different active molecules. The active molecules specifically disclosed herein may be used, as well as any pharmaceutically acceptable salts thereof.

As noted, pharmaceutically acceptable salts of the compounds set out herein below are also contemplated for use in this invention. Such salts are prepared by treating the free acid with an appropriate amount of a pharmaceutically acceptable base. Representative pharmaceutically acceptable bases are ammonium hydroxide, sodium hydroxide, potassium hydroxide, lithium hydroxide, calcium hydroxide, magnesium hydroxide, ferrous hydroxide, zinc hydroxide, copper hydroxide, aluminum hydroxide, ferric hydroxide, isopropylamine, trimethylamine, diethylamine, triethylamine, tripmpylamine, ethanolamine, 2-dimethylaminoethanol, 2-diethylaminoethanol, lysine, arginine, histidine, and the like. The reaction is conducted in water, alone or in combination with an inert, water-miscible organic solvent, at a temperature of from about 0°C to about 100°C, preferably at room temperature. The molar ratio of compounds of structural formula (I) to base used are chosen to provide the ratio desired for any particular salts. For preparing, for example, the ammonium salts of the free acid starting material-a particular preferred embodiment-the starting material can be treated with approximately one equivalent of pharmaceutically acceptable base to yield a neutral salt. When calcium salts are prepared, approximately one-half a molar equivalent of base is used to yield a neutral salt, while for aluminum salts, approximately one-third a molar equivalent of base will be used.

Similarly, salts of aliphatic and/or aromatic amines are also contemplated for use in this invention. A variety of pharmaceutically acceptable salts may be prepared by any of several methods well known to those skilled in the art. Such methods include treatment of a free aliphatic or aromatic amine with an appropriate carboxylic acid, mineral acid, or alkyl halide, or by conversion of the ammonium salt to another form using ion exchange resins.

Compounds prepared in accordance with the design and synthesis methods of this invention are especially attractive because they may preferably be further optimized by incorporation of substituents on either the active molecule and/or the linking group. These latter modifications can also preferably be accomplished using the combinatorial methods disclosed herein.

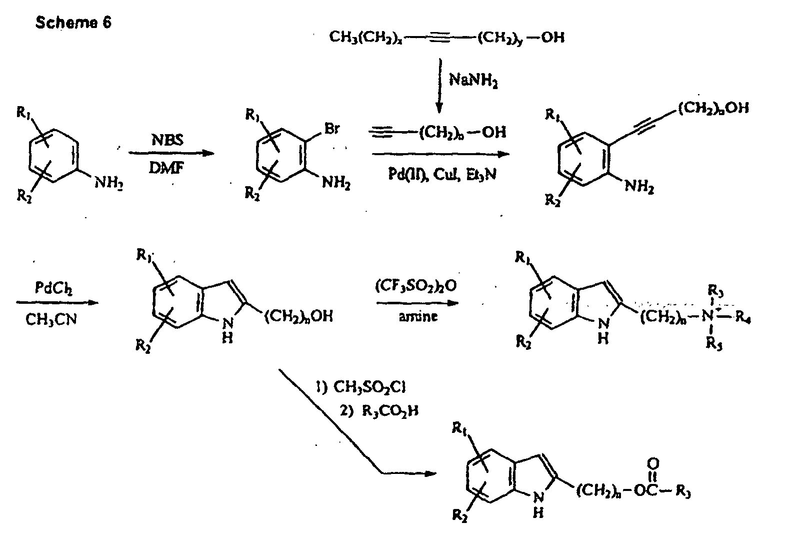

In yet another preferred embodiment, the invention provides a method of making a NAD synthetase inhibitor compound from the route set out in Scheme 6 above, comprising the steps of:
a. brominating an aniline with N-bromosuccinimide to form a 2-bromo-R¹-substituted-aniline or a 2-bromo-R²-substituted-aniline;
b. reacting the 2-bromo-R¹-substituted-aniline or a 2-bromo-R²-substituted-aniline using a Heck coupling reaction to form an alkyne-substituted aniline;
c. reacting the alkyne-substituted aniline using a cyclization reaction to form an indole alcohol;
d. quaternizing the indole alcohol with an amine;
e. reacting the indole alcohol with triflouromethylsulfonic anhydride to provide a triflate; and
f. reacting the indole triflate with an amine to form an indole alkylammonium product.

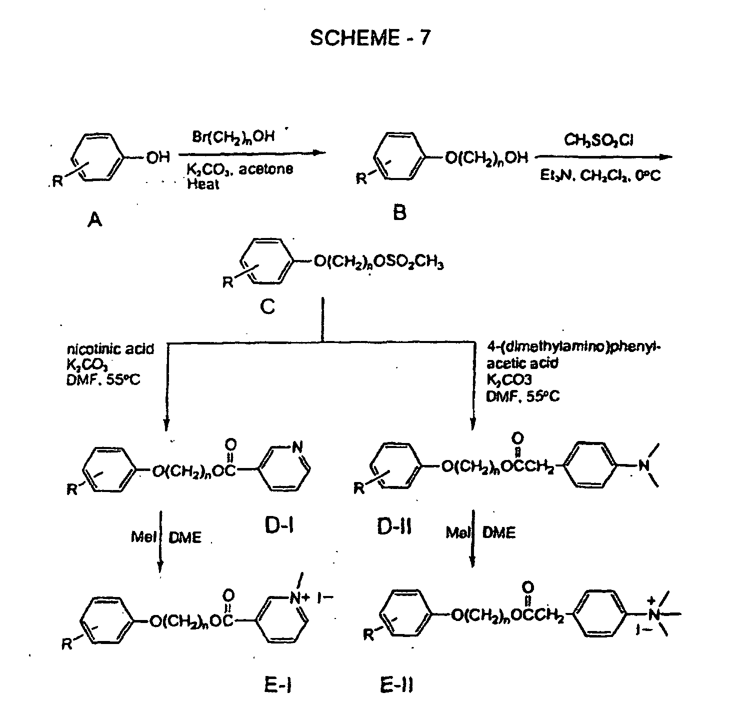

In a preferred embodiment, the invention provides a method of synthesizing a NAD synthetase inhibitor compound from the route set out in Scheme 7 above, comprising the steps of:
a. alkylating a phenol with 7-bromo-1-heptanol to provide 7-(phenyloxy)-1-heptanol;
b. mesylating 7-(phenyloxy)-1-heptanol to provide 7-(phenyloxy)-1-heptyl methanesulfonate;
c. esterifying 7-(phenyloxy)-1-heptyl-methanesulfonate to provide 7-(phenyloxy)-1-heptyl nicotinate; and
d. n-methylating 7-(phenyloxy)-1-heptyl nicotinate to provide [7-(phenyloxy)-1-heptyl-(N-methyl) nictotinate] iodide.

In a further embodiment, the invention provides a compound of Structure 7: 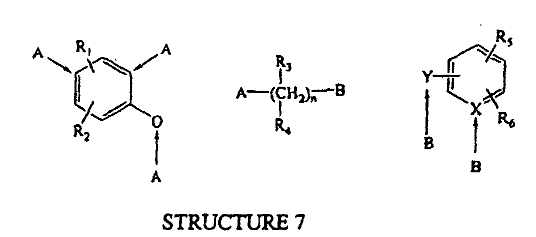 wherein:
X is C or N, Y is C, N, O, S, carboxy, ester, amide or ketone, A and B represent the respective sites of attachment for a linker,
n is an integer of from 1 to 12,
R₁-R₆ each, independently, is H, aryl, hydroxy, ketone, nitro, amino, amidino, guanidino, carboxylate, amide, sulfonate, halogen, a substituted or unsubstituted branched, un-branched or cyclic aliphatic group,
the linker is a branched, un-branched or cyclic aliphatic group,
   wherein:
   aliphatic groups include alkyl, alkenyl and alkynyl groups,
   the linker can further include heteroatoms, and
   when R₁-R₆ represents a cyclic group, the group can include one or more closed rings.
Note that n may also be an integer of from 3 to 10, more preferably 5 to 9, and still more preferably, 6 to 9. The tethered active molecule, e.g., in this example denoted "aryl," moieties may be the same or different.

In further embodiments, the compounds of the present invention preferably correspond to compounds of the Structure 202A.

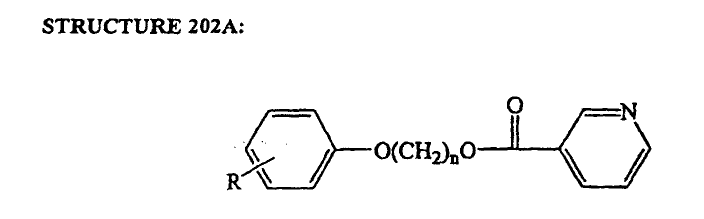

In further embodiments, the compounds of the present invention preferably correspond to compounds of the Structure 202A wherein n is an integer of from 1 to 12, more preferably, from 3 to 10, more preferably from 5 to 9 and, still more preferably from 6 to 9 and wherein R is H; 4-NO₂; 2-CONHPh; 2-NO₂; 4-[1'(4'-acetylpiperazine)]; 2-COCH₃; 3-OCOCH₃; 3-OCH,; 4-COCH₃; 3-OCOPh; 2-CONH₂; 4-CH=CHCOCH₃; 4-OCOPh; 4-CH=CHCOPh; 4-{CO-3'[2'-butylbenzo(b)furan]}; 3-NO₂; 4-[5'-(5'-phenylhydantoin)]; 2-CH=CHCOPh; 2-OCH₃; 4-COPh; 4-CONH₂; 3-COCH₃; 4-OPh; 4-(N-Phthalimide); 3-(N-Morpholine); 2-(N-pyrrolidine); 2-(N-Morpholine); or 4-OCH₂Ph. Further preferred embodiments of the compounds corresponding to Structure 202 are set out in Table 202.

**TABLE 202:**

| **COMPOUNDS 991-1021 CORRESPONDING TO STRUCTURE 202A** | | | |
|---|---|---|---|
| R= | n=4 | n=7 | n=8 |
| H | 991 | 993 | |
| 4-NO, | 992 | 994 | 995 |
| 2-CONHPh | | | 996 |
| 2-NO₂ | | | 997 |
| 4-[1'(4'-acetylpiperazine)] | | | 998 |
| 2-COCH₃ | | | 999 |
| 3-OCOCH₃ | | | 1000 |
| 3-OCH₃ | | | 1001 |
| 4-COCH₃ | | | 1002 |
| 3-OCOPh | | | 1003 |
| 2-CONH₂ | | | 1004 |
| 4-CH=CHCOCH₃ | | | 1005 |
| 4-OCOPh | | | 1006 |
| 4-CH=CHCOPh | | | 1007 |
| 4-{CO-3'[2'-butylbenzo(b)furan]} | | | 1008 |
| 3-NO₂ | | | 1009 |
| 4-[5'-(5'-phenythydantoin)] | | | 1010 |
| 2-CH=CHCOPh | | | 1011 |
| 2-OCH₃ | | | 1012 |
| 4-COPh | | | 1013 |
| 4-CONH₂ | | | 1014 |
| 3-COCH₃ | | | 1015 |
| 4-OPh | | | 1016 |
| 4-(N-phthalimide) | | | 1017 |
| 3-(N-morpholine) | | | 1018 |
| 2-(N-pyrrolidine) | | | 1019 |
| 2-(N-morpholine) | | | 1020 |
| 4-OCH₂Ph | | | 1021 |

In further embodiments, the compounds of the present invention preferably correspond to compounds of the Structure 204A wherein n is an integer of from I to 12, more preferably, from 3 to 10, more preferably from 5 to 9 and, still more preferably, from 6 to 9 and wherein R is 4-NO₂; 2-CONHPh; 2-NO₂; 4-[1'(4'-acetylpiperazine)]; 2-COCH₃; 3-OCOCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-butylbenzo(b)furan]}; 3-NO₂; 4-[5'-(5'-phenylhydantoin)]; 2-CH=CHCOPh; 2-OCH₃; 4-COPh; 4-CONH₂; 3-COCH,; 4-OPh; 4-(N-phthalimide); 3-(N-morpholine); 2-(N-pyrrolidine); 2-(N-morpholine); or 4-OCH₂Ph. Further preferred embodiments of the compounds corresponding to Structure 204 are set out in Table 204.

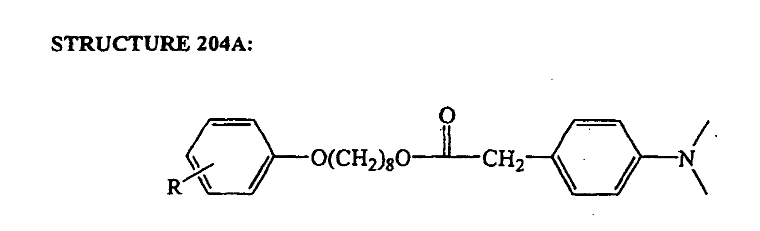

**TABLE 204:**

| **COMPOUNDS 1022-1048 CORRESPONDING TO STRUCTURE 204A** | |
|---|---|
| R= | |
| 4-NO₂ | 1022 |
| 2-CONHPh | 1023 |
| 2-NO₂ | 1024 |
| 4-[1'(4'-acetylpiperazine)] | 1025 |
| 2-COCH₃ | 1026 |
| 3-OCOCH₃ | 1027 |
| 3-OCH₃ | 1028 |
| 4-COCH₃ | 1029 |
| 3-OCOPh | 1030 |
| 2-CONH₂ | 1031 |
| 4-CH=CHCOCH₃ | 1032 |
| 4-OCOPh | 1033 |
| 4-CH=CHCOPh | 1034 |
| 4-{CO-3'[2'-butylbenzo(b)furan]} | 1035 |
| 3-NO₂ | 1036 |
| 4-[5'-(5'-phenylhydantoin)] | 1037 |
| 2-CH=CHCOPh | 1038 |
| 2-OCH₃ | 1039 |
| 4-COPh | 1040 |
| 4-CONH₂ | 1041 |
| 3-COCH₃ | 1042 |
| 4-OPh | 1043 |
| 4-(N-phthalimide) | 1044 |
| R= | |
| 3-(N-morpholine) | 1045 |
| 2-(N-pyrrolidine) | 1046 |
| 2-(N-morpholine) | 1047 |
| 4-OCH₂Ph | 1048 |

In further embodiments, the compounds of the present invention preferably correspond to compounds of the Structure 206 wherein n is an integer of from 1 to 12, more preferably, from 3 to 10, more preferably from 5 to 9 and, still more preferably, from 6 to 9 and wherein R is H; 4-NO₂; 2-CONHPh; 2-NO₂; 2-COCH,; 3-OCH₃; 4-COCH,; 3-OCOPh; 2-CONH₂; 4-CH=CHCOCH₃; 4-OCOPh; 4-CH=CHCOPh; 4-{CO-3'[2'-butylbenzo(b)furan]}; 3-NO₂; 2-CH=CHCOPh; 2-OCH₃; 4-COPh; 3-COCH,; 4-OPh; 4-(N-phthalimide); or 4-OCH₂Ph. Further preferred embodiments of the compounds corresponding to Structure 206 are set out in Table 206.

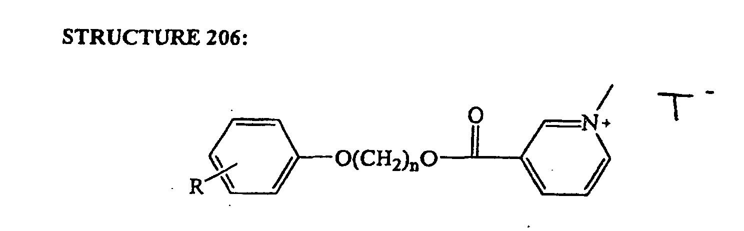

**TABLE 206:**

| **COMPOUNDS 1049-1068 CORRESPONDING TO STRUCTURE 206** | | | |
|---|---|---|---|
| R= | n=4 | n=7 | n=8 |
| H | 1049 | 1051 | |
| 4-NO₂ | 1050 | 1052 | 1053 |
| 2-CONHPh | | | 3054 |
| 2-NO₂ | | | 1055 |
| 2-COCH₃ | | | 1056 |
| 3-OCH₃ | | | 1057 |
| 4-COCH₃ | | | 1058 |
| 3-OCOPh | | | 1059 |
| 2-CONH₂ | | | 1060 |
| 4-CH=CHCOCH₃ | | | 1061 |
| 4-OCOPh | | | 1062 |
| 4-CH=CHCOPh | | | 1063 |
| 4-{CO-3'[2'-butylbenzo(b)furan]} | | | 1064 |
| 3-NO₂ | | | 1065 |
| 2-CH=CHCOPh | | | 1066 |
| 2-OCH₃ | | | 1067 |
| 4-COPh | | | 1068 |
| 3-COCH₃ | | | 1069 |
| 4-OPh | | | 1070 |
| 4-(N-phthalimide) | | | 1071 |
| 4-OCH₂Ph | | | 1072 |

In further embodiments, the compounds of the present invention preferably correspond to compounds of the Structure 208 wherein n is an integer of from 1 to 12, more preferably, from 3 to 10, more preferably from 5 to 9 and, still more preferably, from 6 to 9 and wherein R is 4-NO₂; 2-CONHPh; 2-NO₂; 2-COCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-butylbenzo(b)furan]}; 3-NO₂; 2-CH=CHCOPh; 2-OCH₃; 4-COPh; 3-COCH₃; 4-OPh; 4-(N-mhthalimide); 3-(N-morpholine); 2-(N-morpholine); or 4-OCH₂Ph. Further preferred embodiments of the compounds corresponding to Structure 208 are set out in Table 208.

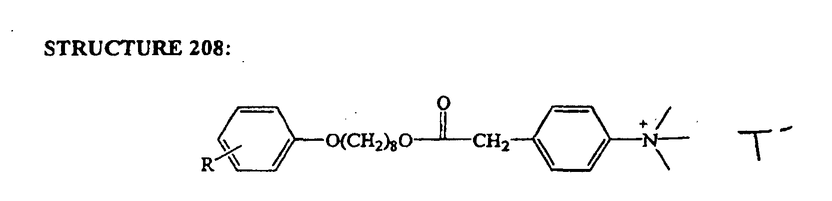

**TABLE 208:**

| **COMPOUNDS 1073-1094 CORRESPONDING TO STRUCTURE 208** | |
|---|---|
| R= | |
| 4-NO₂ | 1073 |
| 2-CONHPh | 1074 |
| 2-NO₂ | 1075 |
| 2-COCH₃ | 1076 |
| 3-OCH₃ | 1077 |
| 4-COCH₃ | 1078 |
| 3-OCOPh | 1079 |
| 2-CONH₂ | 1080 |
| 4-CH=CHCOCH₃ | 1081 |
| 4-OCOPh | 1082 |
| 4-CH=CHCOPh | 1083 |
| 4-{CO-3'[2'-butylbenzo(b)furan]} | 1084 |
| 3-NO₂ | 1085 |
| 2-CH=CHCOPh | 1086 |
| 2-OCH₃ | 1087 |
| 4-COPh | 1088 |
| 3-COCH₃ | 1089 |
| 4-OPh | 1090 |
| 4-(N-phthalimide) | 1091 |
| 3-(N-morpholine) | 1092 |
| 2-(N-morpholine) | 1093 |
| 4-OCH₂Ph | 1094 |

In further embodiments, the compounds of the present invention preferably correspond to compounds of the Structure 210 wherein R is NH₂; NMe₂; NMe₃.I; NH₂.HCl; NMe₂.HCl. Further preferred embodiments of the compounds corresponding to Structure 210 are set out in Table 210.

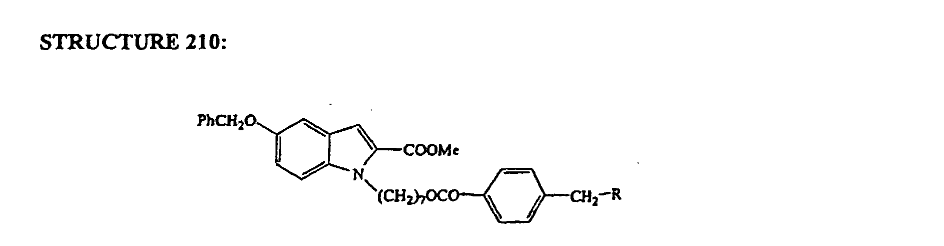

**TABLE 210:**

| **COMPOUNDS 1095-1099 CORRESPONDING TO STRUCTURE 210** | |
|---|---|
| R= | |
| NH₂ | 1095 |
| NMe₂ | 1096 |
| NMe₃.I- | 1097 |
| NH₂.HCl | 1098 |
| NMe₂.HCl | 1099 |

In further embodiments, the compounds of the present invention preferably correspond to compounds of the Structure 212 wherein R' is PhCONH or Ph₃C and R" is H or COOCH₃. Further preferred embodiments of the compounds corresponding to Structure 212 are set out in Table 212.

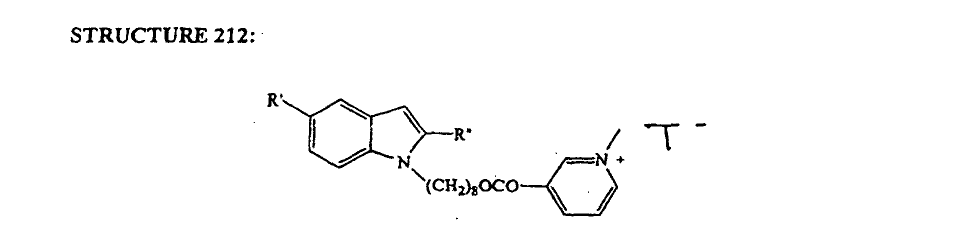

**TABLE 212:**

| **COMPOUNDS 1100-1101 CORRESPONDING TO STRUCTURE 212** | | |
|---|---|---|
| R'= | R"= | |
| PhCONH | H | 1100 |
| Ph₃C | COOCH₃ | 1101 |

In further embodiments, the compounds of the present invention preferably correspond to compounds of the Structure 214 wherein R is 4-hydroxyphenyl or 3-hydroxy-4-methylphenyl. Further preferred embodiments of the compounds corresponding to Structure 214 are set out in Table 214.

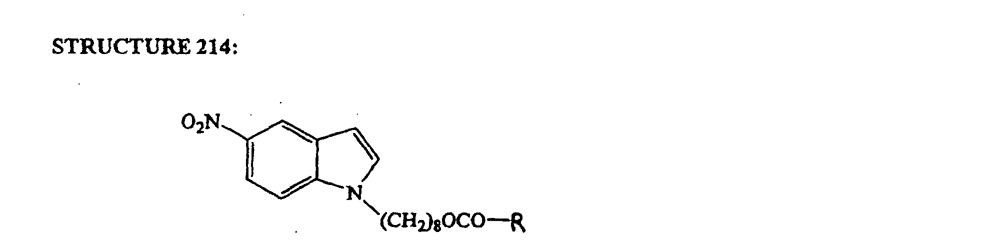

**TABLE 214:**

| **COMPOUNDS 1102-1103 CORRESPONDING TO STRUCTURE 214** | |
|---|---|
| R= | |
| 4-hydroxyphenyl | 1102 |
| 3-hydroxy-4-methylphenyl | 1103 |

In further embodiments, the compounds of the present invention preferably correspond to compounds of Structure 216 wherein R' is PhCONH and and R'' is H or COOCH₃ and n= 7 or 8. Further preferred embodiments of the compounds corresponding to Structure 216 are set out in Table 216.

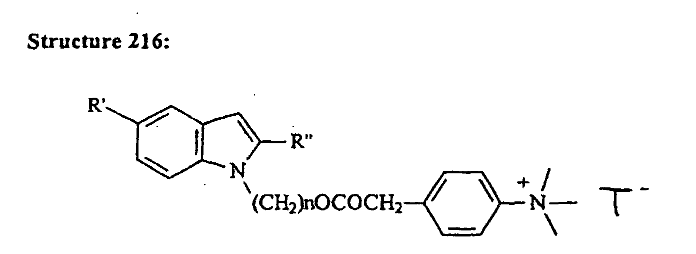

**TABLE 216:**

| **COMPOUNDS 1104-1105 CORRESPONDING TO STRUCTURE 216** | | | |
|---|---|---|---|
| R'= | R"= | n= | |
| PhCONH | H | 8 | 1104 |
| PhCH₂O | COOCH₃ | 7 | 1105 |

In a particularly preferred embodiment of the invention herein, the present invention comprises compounds of the structures in Table 301 below.

In a further preferred embodiment, the present invention comprises one or more compounds from Table 303, below.

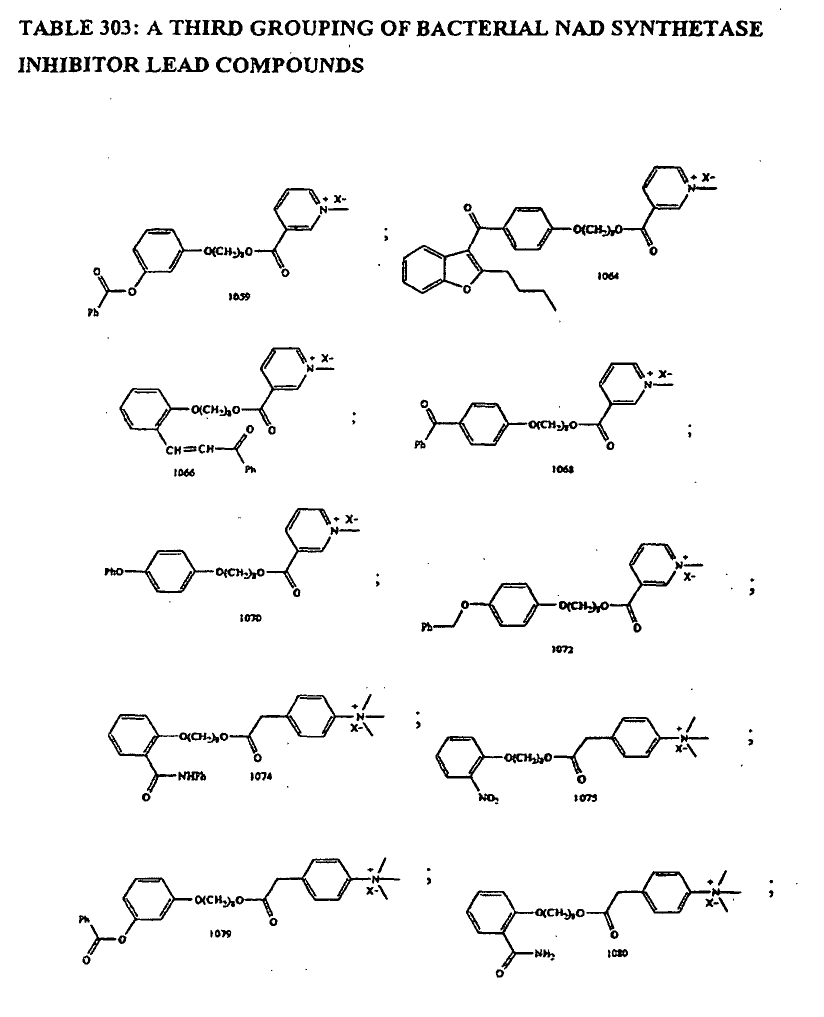

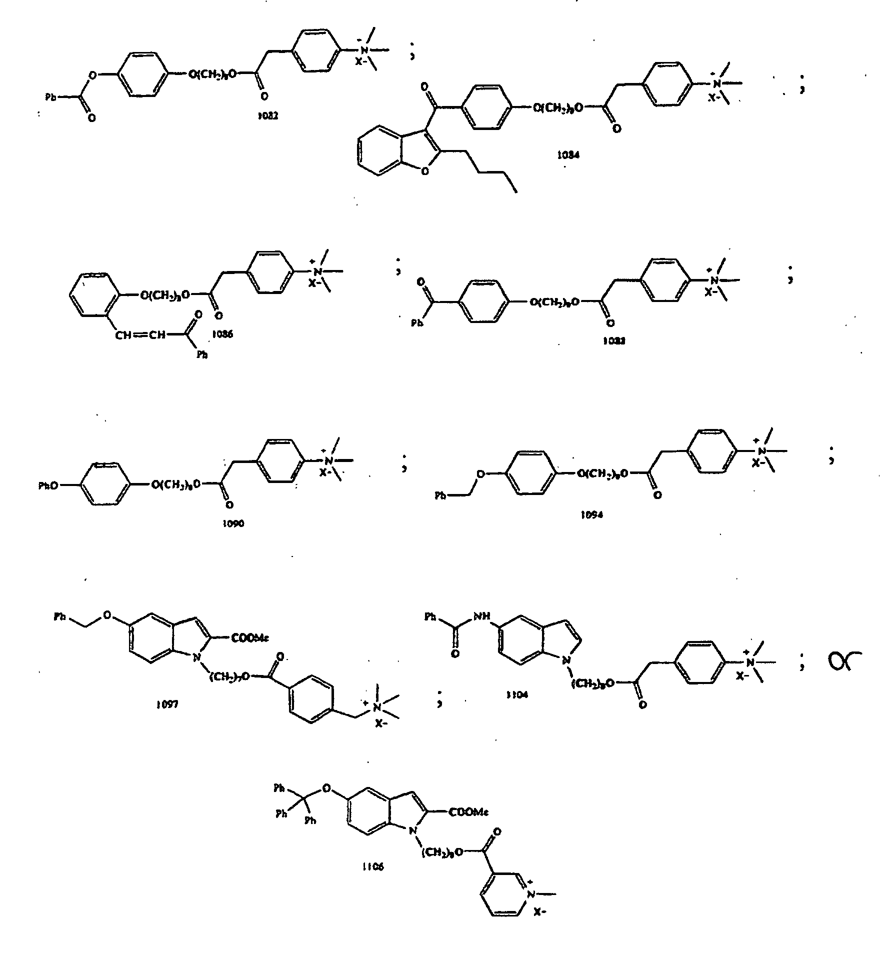

The compounds of the invention may be readily synthesized using techniques generally known to synthetic organic chemists. Suitable experimental methods for making and derivatizing aromatic compounds are described, for example, methods for making specific and preferred compounds of the present invention are described in detail in Examples 1 to 4 below.

Reference is made further to a method of generating a library comprising at least one bacterial NAD synthetase enzyme inhibitor compound comprising the steps of:
a. obtaining the crystal structure of a bacterial NAD synthetase enzyme;
b. identifying one or more sites of catalytic activity on the NAD synthetase enzyme;
c. identifying the chemical structure of the catalytic sites on the NAD synthetase enzyme;
d. selecting one or more active molecule compounds that will demonstrate affinity for at least one of the catalytic sites on the NAD synthetase enzyme;
e. synthesizing one or more dimeric compounds comprised of at least one active molecule wherein the active molecule compound are joined by means of n linker compounds and wherein n is an integer of from 1 to 12, and
f. screening the one or more compounds for NAD synthestase inhibitor activity.

The library further comprises one or more compounds set forth in Table 301 above. In one embodiment, a library of compounds according to the invention herein preferably includes compounds of the structures set out in structures 1 to 1106 above. Further preferably, the library comprises a compound of Structure 2, still preferably, Structure 4, further preferably, Structure 6, and further preferably, Structure 7. In further preferred embodiments, the library comprises at least one compound of Structure 8, Structure 10, Structure 12, Structure 16 or Structure 18.

There is also the possibility of one or more dimeric compounds comprising at least two active molecules. Still preferably, the active molecules are the same. Alternatively, it is preferable that the active molecules are different.

A software program that predicts the binding affinities of molecules to proteins is utilized in the active molecule selection step. Further preferably, a software program that evaluates the chemical and geometric complementarity between a small molecule and macromolecular binding site is utilized in the active molecule selection step.

In yet another preferred embodiment, the compounds are synthesized utilizing a rapid, solution phase parallel synthesis and wherein the compounds are generated in a combinatorial fashion.

In a preferred embodiment, the invention provides a method of treating or preventing a microbial infection in a mammal comprising administering to the mammal a treatment effective or treatment preventive amount of a bacterial NAD synthetase enzyme inhibitor compound. In a particularly preferred embodiment, the compound administered in the method is a compound as set out previously in Table 301. In another embodiment, invention herein preferably includes compounds 1 to 1106 above. Further preferably, the compound administered comprises at least one compound of Structure 2, still preferably, Structure 4, further preferably, Structure 6. In further preferred embodiments, the compounds administered in the method comprise compounds of Structure 8, Structure 10, Structure 12, Structure 16 or Structure 18.

In a preferred embodiment, the invention provides administering a broad spectrum antibiotic to a mammal in need of such treatment or prevention. In a further preferred embodiment, the microbial infection is a bacterial infection. In yet another embodiment of the invention, the bacterial infection is caused by a bacterium that is a gram negative or gram positive bacteria. The bacterial infection may preferably be caused by an antibiotic resistant strain of bacteria.

Further provided by the invention herein is preferably a method of killing a prokaryote with an amount of prokaryotic NAD synthetase enzyme inhibitor compound to reduce or eliminate the production of NAD whereby the prokaryote is killed. A method of decreasing prokaryotic growth, comprising contacting the prokaryote with an amount of a prokaryotic NAD synthetase enzyme inhibitor effective to reduce or eliminate the production of NAD whereby prokaryotic growth is decreased is also provided. In the method of killing a prokaryote, as well as in the method of decreasing prokaryotic growth, the compound comprises one or more compounds of Table 301, Table 302 or Table 303. Still preferably, the invention comprises one or more of compounds 1 to 1106 above. Further preferably, the compound administered is a compound of Structure 2, still preferably, a compound of Structure 4, further preferably, Structure 6. In further preferred embodiments, the compounds administered in the methods compounds of Structure 7, Structure 8, Structure 10, Structure 12, Structure 16 or Structure 18.

In the method of killing a prokaryote, as well as in the method of decreasing prokaryotic growth, the prokaryote is a bacterium. Further preferably, the bacterium is a gram negative or a gram positive bacteria. Still preferably, the prokaryote is an antibiotic resistant strain of bacteria.

Also in the method of killing a prokaryote, as well as in the method of decreasing prokaryotic growth, the NAD synthetase enzyme inhibitor is a compound that selectively binds with catalytic sites or subsites on a bacterial NAD synthetase enzyme to reduce or eliminate the production of NAD by the bacteria.

In the methods discussed above, the compound is preferably administered by oral, rectal, intramuscular, intravenous, intravesicular or topical means of administration. The compounds of this invention can be administered to a cell of a subject either *in vivo* or *ex vivo*. For administration to a cell of the subject *in vivo*, as well as for administration to the subject, the compounds of this invention can be administered orally, parenterally (*e*.*g*., intravenously), by intramuscular injection, by intraperitoneal injection, subcutaneous injection, transdermally, extracorporeally, topically, mucosally or the like.

Depending on the intended mode of administration, the compounds of the present invention can be in pharmaceutical compositions in the form of solid, semi-solid or liquid dosage forms, such as, for example, tablets, suppositories, pills, capsules, powders, liquids, suspensions, lotions, creams, gels, or the like, preferably in unit dosage form suitable for single administration of a precise dosage. The compositions will include, as noted above, an effective amount of the selected composition, possibly in combination with a pharmaceutically acceptable carrier and, in addition, may include other medicinal agents, pharmaceutical agents, carriers, adjuvants, diluents, etc.

Parenteral administration of the compounds of the present invention, if used, is generally characterized by injection. Injectables can be prepared in conventional forms, either as liquid solutions or suspensions, solid forms suitable for solution of suspension in liquid prior to injection, or as emulsions. As used herein, "parenteral administration" includes intradermal, subcutaneous, intramuscular, intraperitoneal, intravenous and intratracheal routes. One approach for parenteral administration involves use of a slow release or sustained release system such that a constant dosage is maintained. *See e*.*g*., U.S. Patent No. 3,610,795, which is incorporated by reference herein. These compounds can be present in a pharmaceutically acceptable carrier, which can also include a suitable adjuvant. By "pharmaceutically acceptable," it is meant a material that is not biologically or otherwise undesirable, *i*.*e*., the material may be administered to an individual along with the selected compound without causing substantial deleterious biological effects or interacting in a deleterious manner with any of the other components of the composition in which it is contained.

Routes of administration for the compounds herein are preferably in a suitable and pharmacologically acceptable formulation. When administered to a human or an animal subject, the bacterial NAD synthetase enzyme inhibitor compounds of the libraries herein are preferably presented to animals or humans orally, rectally, intramuscularly, intravenously, intravesicularly or topically (including inhalation). The dosage preferably comprises between about 0.1 to about 15g per day and wherein the dosage is administered from about 1 to about 4 times per day. The preferred dosage may also comprise between 0.001 and 1 g per day, still preferably about 0.01, 0.05, 0.1, and 0.25, 0.5, 0.75 and 1.0 g per day. Further preferably, the dosage may be administered in an amount of about 1, 2.5, 5.0, 7.5,10.0, 12.5 and 15.0 g per day. The dosage may be administered at a still preferable rate of about 1, 2, 3, 4 or more times per day. Further, in some circumstances, it may be preferable to administer the compound of the invention continuously, as with, for example, intravenous administration. The exact amount of the compound required will vary from subject to subject, depending on the species, age, weight and general condition of the subject, the particular compound used, its mode of administration and the like. Thus, it is not possible to specify an exact amount for every compound. However, an appropriate amount can be determined by one of ordinary skill in the art using only routine experimentation given the teachings herein.

If *ex vivo* methods are employed, cells or tissues can be removed and maintained outside the subject's body according to standard protocols well known in the art. The compounds of this invention can be introduced into the cells via known mechanisms for uptake of small molecules into cells (*e*.*g*., phagocytosis, pulsing onto class I MHC-expressing cells, liposomes, etc.). The cells can then be infused (*e*.*g*., in a pharmaceutically acceptable carrier) or transplanted back into the subject per standard methods for the cell or tissue type. Standard methods are known for transplantation or infusion of various cells into a subject.

It is further provided a method of disinfecting a material contaminated by a microbe, comprising contacting a contaminated material with a bacterial NAD synthetase enzyme inhibitor compound in an amount sufficient to kill or deactivate the microbe. In yet another embodiment, the compound utilized for contacting comprises one or more compounds of Table 301, Table 302 or Table 303. The compounds utilized for contacting may also comprise one or more of compounds 1 to 1106. Further preferably, the compound utilized for contacting is a compound of Structure 2, still preferably, a compound of Structure 4, further preferably, Structure 6. In further preferred embodiments, the compounds utilized for contacting in the method comprise compounds of Structure 7, Structure 8, Structure 10, Structure 12, Structure 16 or Structure 18.

In yet a further embodiment of the invention herein, the compounds of the present invention are effective as disinfectant materials for, for example, hard or soft surfaces, fabrics, and other contaminated materials such as those in hospitals, households, schools, nurseries, and any other location. In yet another embodiment, the invention provides a method for disinfecting comprising contacting a bacterial contaminated material with a bacterial NAD synthetase enzyme inhibitor compound.

In a further aspect of the invention, an *in vitro* "one-at-a-time" method of screening compounds for bacterial NAD synthetase enzyme inhibitory activity is provided. In a preferred embodiment, this *in vitro* method of screening compounds for such activity comprises the steps of preparing a solution comprising pure bacterial NAD synthetase enzyme, contacting the solution with the compounds set out herein, and determining the rate of the enzyme-catalyzed reaction. Preferably, measurement of the rate of enzyme-catalyzed reaction comprises a measure of NAD synthetase inhibitory activity. In a further embodiment, the rate of enzyme-catalyzed reaction comprises a measure of antibacterial activity. In a still further embodiment, the rate of enzyme-catalyzed reaction corresponds to a measure of antimicrobial activity.

Preferably, the method of preparing the bacterial enzyme solution for use in the *in vitro* screening method comprises utilizing molecular biological methods to over-express bacterial NAD synthetase enzyme, for example from *B. subtilis*, in *E. coli.* One of skill in the art will recognize techniques useful for such a process. A particularly preferable method comprises: a) cloning the Out B gene encoding NAD synthetase enzyme and over-expressing the gene in *E. coli*; b) purifying the cloned and over-expressed gene by ion-exchange; c) purifying further the enzyme material from step b using ion-exchange methods; d) further purifying the material from step c using size exclusion chromatography wherein the bacterial NAD synthetase enzyme is essentially pure; and e) preparing an assay solution in quantities of about 10 to 15 mg pure bacterial NAD synthetase enzyme per liter of fermentation broth. As used herein, "essentially pure" means greater than about 90% purity, more preferably, greater than about 95% purity and, still more preferably, greater than about 99% purity.

In one embodiment of the *in vitro* screening method, the following procedure is utilized to measure the rate of enzyme catalyzed reaction. A solution of HEPPS, pH 8.5, with KCl is prepared containing the following species: ATP, NaAD, MgCl₂, NH₄Cl, ADH, and ETOH. A stock solution of test inhibitors is then prepared by dissolving solid samples into 100% DMSO. The test compound stock solution is then added to the mixture to give the final test compound concentrations. NAD synthetase enzyme solution is added, the mixture is mixed three times, and the absorbance at 340 nm is then monitored kinetically using an UV-Vis spectrophotometer. The initial kinetics trace after enzyme addition is then fit to a straight line using linear regression, with this rate is then compared to that of a control containing no inhibitor, using the following formula to calculate % Inhibition: {(Vo-V)/Vo}*100%, where Vo is the rate of the reaction with no test compound present and V is the rate of the reaction with test the test compound added. Each compound is tested in triplicate, and the resulting values for % inhibition were averaged to give the listed value. IC₅₀ (concentration needed to inhibit 50% of the test bacteria) values were obtained for select compounds by assaying six different concentrations of test compound, in triplicate, at concentrations between 0.0 and 2.0 mM, and plotting the resulting % inhibition values against the -LOG of the test compound dose to reveal the concentration at which 50% inhibition is observed.

Preferably, the *in vitro* method can also be adapted to allow screening for compounds with bacterial NAD synthetase enzyme inhibitory activity in other forms of bacteria, as well as other types of microbes. For example, the above-described procedure can be adapted to screen for inhibitory activity in at least the following bacteria types:

| **BACTERIUM** | **STRAIN** |
|---|---|
| *Escherichia coli* K-12 | MG1655 |
| | (CGSC#6300) |
| *Escherichia coli* K-12 | W3110 |
| | (CGSC#4474) |
| *Salmonella typhimurium* | LT2 TT366 |
| *Streptococcus pneumonia* | D39 |
| *Streptococcus pneumoniae* | WU2 |
| *Bacillus subtilis* | A700 |

In a further embodiment of the *in vitro* screening method, the method can be used to screen existing compounds *e*.*g*., commercially available compounds, such as 5-nitroindole and N-methyl nicotinic acid. One of skill in the art will recognize the manner in which the designing and screening methods herein can be utilized to identify commercially available compounds, such as the previous non-exhaustive list, that will exhibit NAD synthetase enzyme inhibitory activity, both in bacteria and other microbes.

In order to test a library of NAD synthetase enzyme inhibitor compounds, such as those of the present invention, it is particularly preferable to utilize a method of rapid (high throughput) screening. To this end, the potential inhibitory activity of the library of synthetic compounds in one embodiment is assessed via a coupled enzymatic assay. The coupled assay involves two steps as summarized below.

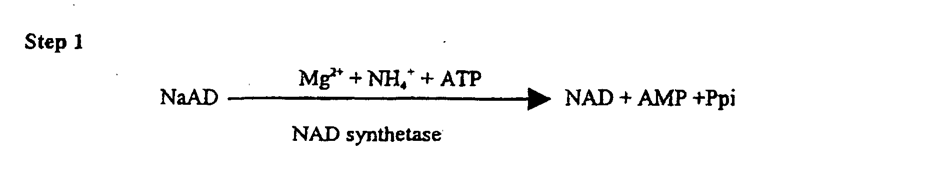

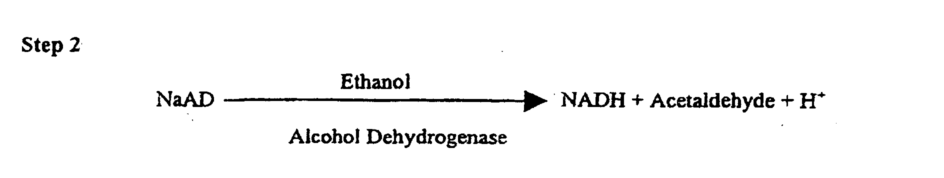

In order to rapidly measure the inhibitory activities of the compounds in the library, the invention provides a high through-put screening system (HTS system). The HTS system preferably utilizes an integrated robotic system that coordinates the functions of a liquid handler and a spectrophotometer. The robotic station is preferably responsible for the movement of all hardware and the integration of multiple stations on the worksurface. The liquid handler is preferably programmed to perform all phases of liquid dispensing arid mixing. The spectrophotometer is preferably equipped to monitor absorbance in a 96-well plate format.

In one embodiment, the assay is designed for a 96-well plate format reaction buffer containing HEPPS buffer, pH 8.5, MgCl₂, NH₄Cl₂, KCl, NaAD, n-Octyl-D-Glucopyranoside, ethanol, NAD synthetase, and yeast alcohol dehydrogenase. At the next stage, the liquid handler dispenses DMSO (with or without inhibitor) into the reaction well. The liquid handler mixes these components utilizing a predefined mixing program. The reaction is initiated by the addition of a solution of ATP dissolved in buffer. The reaction is monitored by measuring the increase in absorbance at 340nm. The linear portion of the reaction is monitored for a period of time. The initial velocity is determined using the software supplied with the spectrophotometer.

The compounds of the library herein are supplied as a stock with a concentration dissolved in 100% DMSO. An initial screen is conducted on all compounds using a 2 or 3 concentration screen. The 2 panel screen used concentrations of 0.2mM and 0.1mM for the compounds. The 3 panel screen used concentrations of 0.2mM, 0.1mM, and 0.05mM. From the initial screen, "lead compounds" *e*.*g*., those compounds which demonstrated the greatest inhibitory capacity, are then preferably subjected to a wider screen of concentrations (0.1mM to 0.001mM) to determine the apparent IC-50 values for each compound.

In still a further preferred embodiment of the invention herein, the high through-put method is utilized to screen commercially available compounds for bacterial NAD synthetase enzyme inhibitory activity. In an additional embodiment, the NAD synthetase enzyme inhibitor compounds are tested as inhibitors of bacterial growth against a variety of bacteria types.

In a further embodiment of the invention, compounds within the libraries of NAD synthetase inhibitor compounds are evaluated for antibacterial and antimicrobial activity. In one embodiment, compounds are preferably evaluated for their potential to inhibit the growth of *Bacillus subtilis, Pseudomonas aeruginosa*, and *Staphoyloccus epidermitis*. The inhibitors are preferably initially screened in duplicate at one concentration. The test inhibitor compounds are prepared by dissolving the solid samples in DMSO. Aliquots from the inhibitor stocks are placed in sterile 96-well plates by the liquid handler discussed previously. Cultures of *B. subtilis, P. aeruginosa* and *S. epidermitis* are prepared in liquid broth (LB) media and incubated in an orbital shaker overnight. Dilutions (with LB media) of the overnight cultures are added to the 96-well plates containing the inhibitors. The plates are incubated and the absorbance measured at 595nm in a plate reader.

In this embodiment of the invention, a diluted overnight culture without inhibitors serves as one of three controls in the experiments. A positive control, which includes an identical concentration of the drug Tobramycin as the inhibitors being tested, and a DMSO control are also performed during each inhibitor screen. The DMSO control was included for comparison with the control that contained no inhibitors.

Percent inhibition of each inhibitor was calculated by the following formula: {(A_{D} - A₁)/ A_{D}}*100; where A_{D} = the absorbance at 595nm of the DMSO control and A₁ = the absorbance of the inhibitor at 595nm.

In a further embodiment, dose responses are performed on the compounds that inhibited greater than 85% in the initial screen. The dose responses consisted of 5 different concentrations (from 100 mM - 0.1 mM) of each inhibitor and the positive control Tobramycin. The cultures are prepared and grown in the same manner as the inhibitor screens and the same controls were included. The absorbance is measured every hour and a half during the six hours of growth. Percent inhibitions are calculated again for each concentration tested. The lowest concentration that resulted in an 85% inhibition or higher is termed the Minimum Inhibitory Concentration that inhibited bacterial growth 85% (MIC₈₅).

When a NAD synthetase enzyme inhibitor compound of a library herein are to be administered to a humans or an animal *e*.*g*., a mammal, it is preferable that the compounds show little or no toxicity to the patient. Therefore, in one embodiment of the invention herein, the toxicities of the NAD-synthetase enzyme inhibitors are evaluated using human epithelial cells as set out in Example 11 below.

### EXAMPLES

The following examples are set forth so as to provide those of ordinary skill in the art with a complete disclosure and description of how the compositions and methods claimed herein are made and evaluated, and are intended to be purely exemplary of the invention and are not intended to limit the scope of what the inventors regard as their invention. Efforts have been made to ensure accuracy with respect to numbers (*e*.*g*., amounts, temperature, etc.) but some errors and deviations should be accounted for. Unless indicated otherwise, parts are parts by weight, temperature is in °C or is at room temperature, and pressure is at or near atmospheric.

### EXAMPLE 4: GENERAL EXPERIMENTAL PROCEDURES USED FOR PREPARING SOLUTION PHASE COMBINATORIAL LIBRARIES DESCRIBED IN SCHEME 6

The following Example 4 describes a preferred embodiment of the invention herein for compounds prepared according to the synthetic pathway set out in Scheme 6, described previously. One of skill in the art will recognize that many possible variations on this embodiment exist that will not result in deviation from the novel and unobvious aspects of the invention.
**A. Bromination of anilines.** An anhydrous dimethyl formamide (DMF) solution (40 mL) of a commercially available aniline (0.02 mol) was treated with N-bromosuccinimide (NBS, 1.1 eq.) at room temperature overnight. The resulting mixture was quenched by pouring it onto ice and extracted with ethyl acetate (EtOAc, 2 x 30 mL). The combined organic layers were washed with water (30 mL), brine (30 mL), dried over MgSO₄, filtered and concentrated to give the product.
**B. Heck coupling.** To an anhydrous triethylamine solution (TEA, 3 mL) of 2-bromo-R'-substituted-aniline (0.006 mol) (1 eq), in 10 x 1.3 cm test tubes, was added *bis*-triphenylphosphine palladium chloride (2 mol%) at room temperature followed by the addition of copper iodide (2 mol%). To this heterogeneous mixture, the corresponding terminal alkynol (1.5 eq.) and glass beads were added. The resulting mixture was allowed to react for 6h. at 80°C under vigorous vortex shaking. Upon cooling, the reaction mixture was filtered through a celite bed (in 5 mL disposable syringes). Concentration under high vacuum (speed-vac) afforded the product.
**C. Cyclization to form indoles.** To an anhydrous acetonitrile (3 mL) solution of alkyne-substituted aniline in 10 x 1.3 cm test tubes at room temperature was added palladium chloride (2mol%) followed by the addition of glass beads. The resulting mixture was heated to 60 °C for 1h under vigorous vortex shaking. Upon cooling, the reaction mixture was filtered through a bed of celite (in 5 mL disposable syringes). The solvent was evaporated under high vacuum (speed-vac) to afford the products.
**D. Quaternization with amines.** To a cooled (0 °C) solution of the indole alcohol in aromatic amine (pyridine, quinoline, or isoquinoline) (2 mL), under a nitrogen atmosphere in 10 x 1.3 cm test tubes, was added trifluoromethanesulfonyl anhydride (Tf₂O) (1.3 eq.). The resulting solution was allowed to react for 6 h. The reaction mixture was quenched by the addition of an ice-cold 1.5N HCl solution (3 mL) followed by the addition of EtOAc (4 mL). The organic layer was washed with water (3 mL), brine (3 mL), dried over MgSO₄, and filtered through a silica gel column (1 x 2 cm, in 5 mL syringes) in order to remove unreacted organic materials. The column was then flushed with a dichloromethane:methanol (19:1) solution (4 mL). This extract was concentrated to afford the products.
**E. Formation of isolated mesylate.** To an anhydrous DCM solution (2 mL) of the indole alcohol was added TEA (1.5 eq.) at room temperature in 10 x 1.3 cm test tubes. The resulting solution was cooled to 0 °C and treated with methanesulfonyl chloride (1.1 eq.) for 1 h. The reaction mixture was quenched by the addition of water (3 mL), followed by DCM (3 mL). The organic layer was washed with brine (3 mL), dried over MgSO₄, filtered through a celite bed (in 5 mL disposable syringes) and concentrated under high vacuum (speed-vac) to give the indole mesylates.
**F. Formation of ester.** To an anhydrous DMF solution (2 mL) of the indole mesylate (1 eq.) in 10 x 1.3 cm test tubes was added the corresponding carboxylic acid (R₃-COOH, 2 eq.) followed by K₂CO₃ (2 eq.) and glass beads at room temperature. The resulting suspension was heated to 55 °C for 16 h under vigorous vortex shaking. Upon cooling the reaction mixture was quenched by adding water (3 mL) followed by ethyl acetate (3 mL). The organic layer was washed with brine (4 mL), dried under MgSO₄, filtered through a cotton bed (in 5 mL disposable syringes) and concentrated under high vacuum (speed-vac) to give the final ester.

### EXAMPLE 5: GENERAL EXPERIMENTAL PROCEDURES USED FOR PREPARING LIBRARIES DESCRIBED IN SCHEME 7

### A. EXPERIMENTAL PROCEDURE FOR PREPARING COMPOUNDS SINGLY IN SCHEME 7 (n = 7)

### A. Alkylation of Phenol with 7-bromo-1-heptanol

Phenol (0.098g, 1.04mmol) and 7-bromo-1-heptanol (0.243g, 1.246mmol) were dissolved in acetone (25mL) in a 50mL round bottomed flask. Potassium carbonate (0.86g, 6.23mmol) was added to this solution and the flask was fitted with a condenser and refluxed using an oil bath at 70°C for a period of 26 hours. The reaction flask was then cooled to room temperature. The contents of the flask was then filtered through a fluted filter paper and washed with acetone (10mL). The combined filtrate was then evaporated to dryness under reduced pressure. The residue obtained was dissolved in ethyl acetate (25mL). The ethyl acetate solution was then washed with 1N. NaOH (3 X 5mL), water (2 X 5mL) and brine (2 X 5mL). The organic layer was dried over Na₂SO₄. Removal of solvent from the dried extract under reduced pressure afforded the product. The product was then purified by flash chromatography (Si gel, 12 X 2.5cm) using ethyl acetate : hexane (1:1) to afford the pure 7-(phenyloxy)-1-heptanol (0.22g, quantitative yield). The product alcohol from step A was analyzed yielding the following confirmatory data: ¹H-NMR (CDCl₃) δ 1.31-1.52 (m, 6H), 1.52-1.63 (m, 2H), 1.73-1.89 (m, 2H), 1.97 (bs, 1H), 3.61 (t, 2H, J=6.57Hz), 3.93 (t, 2H, J=6.50Hz), 6.80-6.97 (m, 3H) and 7.20-7.28 (m, 2H); ¹³C-NMR (CDCl₃) δ 25.5, 25.8, 29.0, 29.1, 32.4, 62.5, 67.7, 114.3, 120.3, 129.2 and 158.8; IR (neat): 3348 cm⁻¹; MS (ES⁺): 209 (M+1).

### B. Mesylation of 7-(phenyloxy)-1-heptanol

A solution of 7-(phenyloxy)-1-heptanol (0.21g, 1.01mmol) in anhydrous methylene chloride (20mL), taken in a 50 mL round bottomed flask was cooled to 0°C using an ice bath. Methanesulfonyl chloride (0.177g, 1.55mmol) was added to this followed by a dropwise addition of triethylamine (0.28mL, 2.11 mmol). The reaction mixture was then stirred at 0°C for 1hour and allowed to attain room temperature in 2hours. It was then quenched with 1N. HCl (10mL) and diluted with methylene chloride (20mL). The organic layer was separated and the aqueous layer was extracted with methylene chloride (2X10mL). The combined organic layer was washed with 1N. HCl (3X10mL), water (2X10mL) and brine (2XmL). The organic layer was dried over Na₂SO₄. Removal of solvent from the dried extract under reduced pressure afforded the product 7-(phenyloxy)-1-heptyl methanesulfonate (0.25g, 86.5%). The product from the step B was analyzed yielding the following confirmatory data: ¹H-NMR (CDCl₃) 1.31-1.52 (m, 6H), 1.71-1.84 (m, 4H), 2.98 (s, 3H), 3.94 (t, 2H, J=6.43Hz), 4.21 (t, 2H, J=6.51Hz), 6.80-6.94 (m, 3H) and 7.24-7.30 (m, 2H); ¹³C-NMR (CDCl₃) 25.2, 25.7, 28.6, 28.9, 29.0,.37.1, 67.5, 69.9, 114.3, 120.3, 129.2 and 158.9; IR (neat): 1349 cm⁻¹; MS (ES⁺): 287 (M+1).

### C. Esterification of 7-(phenylouy)-1-heptyl methanesulfonate

A solution of 7-(phenyloxy)-1-heptyl methanesulfonate (0.2g, 0.699mmol) and nicotinic acid (0.173g, 1.41mmol) in anhydrous dimethyl formamide (15mL) was placed in a 25mL round bottomed flask. Potassium carbonate (0.097g, 0.702mmol) was added to this solution and the reaction mixture was heated at 50-55°C using an oil bath for a period of 16 hours. It was then allowed to attain room temperature diluted with ethyl acetate (40mL) and quenched with saturated NH₄Cl containing ice (20mL). The organic layer was separated and the aqueous layer was extracted with ethyl acetate (3X10mL). The combined organic layer was washed with saturated NaHCO₃ (3X 10mL), water (2X10mL) and brine (2X10mL). The organic layer was dried over Na₂SO₄. Removal of solvent from the dried extract under reduced pressure afforded the product 7-(phenyloxy)-1-heptyl nicotinate (0.176g, 80.4%). The product ester from step C was analyzed yielding the following confirmatory data: ¹H-NMR (CDCl₃) δ 1.38-1.62 (m, 6H), 1.76-1.92 (m, 4H), 3.95 (t, 2H, J=6.45Hz), 4.35 (t, 2H, J=6.64Hz), 6.85-6.98 (m, 3H), 7.22-7.38 (m, 2H), 7.38 (dd, 1H, J,=7.93Hz, J₂=4.93Hz), 8.29 (dt, 1H, J₁=7.91Hz, J₂=1.92Hz), 8.77 (dd, 1H, J₁=4.83Hz, J₂=1.70Hz) and 9.23 (d, 1H, J=2.00Hz), ¹³C-NMR (CDCl₃) 8 25.8, 25.9, 28.5, 28.9, 29.1, 65.4, 67.6, 114.3, 120.4, 123.2, 126.2, 129.3, 136.9, 150.8, 153.2, 158.9 and 165.2; IR (neat): 1712 cm⁻¹; MS (ES⁺): 314 (M+1); Anal. Calcd. for C₁₉H₂₃NO₃: C, 72.80; H, 7.40 and N, 4.47. Found: C, 72.94; H, 7.54 and N, 4.51.

### D. N-Methylation of 7-(phenyloxy)-1-heptyl nicotinate

7-(phenyloxy)-1-heptyl nicotinate (0.05g, 0.159mmol) was dissolved in anhydrous dimethoxyethane (5mL) taken in a 10mL round bottomed flask fitted with a condenser. Methyl iodide (0.2ml, 3.21mmol) was added to this and heated at reflux for 16 hours. It was then allowed to attain room temperature. The solid product formed was filtered, washed with hexanes and dried to obtain the product [7-(phenyloxy)-1-heptyl (N-methyl) nicotinate] iodide (.04g, 55.3%). The product from the step D was analyzed yielding the following confirmatory data. mp. 82°C; ¹H-NMR (MeOH-d₄) δ 1.41-1.58 (m, 6H), 1.76-1.88 (m, 4H), 3.96 (t, 2H, J=6.54Hz), 4.47 (t, 2H, J=6.74), 4.51 (s, 3H), 6.82-6.91 (m, 3H), 7.21-7.27 (m, 2H), 8.22 (dd, 1H, J₁=7.89Hz, J₂=6.31Hz), 9.03 (d, 1H, J=8.10Hz), 9.12 (d, 1H, J=6.09Hz) and 9.48 (s, 1H)); ¹³C-NMR (MeOH-d₄) δ 27.1, 27.2, 29.6, 30.2, 30.4, 68.3, 68.9, 115.6, 121.6, 129.4, 130.5, 132.3, 146.4, 148.2, 149.8, 160.67 and 162.9; IR (neat): 1719cm⁻¹; MS (ES⁺): 328 (M+); Anal. Calcd. for C₂₀H₂₆NO₃I: C, 52.74; H, 5.76; N, 3.08. Found: C, 52.51; H, 5.77; N, 2.96.

### B. GENERAL EXPERIMENTAL PROCEDURES USED FOR PREPARING SOLUTION PHASE COMBINATORIAL LIBRARIES DESCRIBED IN SCHEME 7 (n = 8)

### A. General considerations

The solvents used were puchased as anhydrous in Sure-Seal™ bottles from Aldrich chemical company. The starting phenols (A) and reagents were purchased from Aldrich, Lancaster or Acres chemical companies and used as such. Reactions were carried out in an orbital shaker purchased from Digi-Block laboratory devices company. Evaporation of solvents were carried out in 25ml wide mouthed vials using a Savant SC210 speedvac plus instrument. Parallel filtrations were carried out using a Burdick & Jackson 24-port manifold. Preparative parallel chromatography were performed on Baker flash silica gel (40µ) packed in 5ml plastic disposable syringes.

### B. Synthesis of alcohols (B)

To the solution of commercially available phenols (**A**) (1-1.5mmol) and 8-bromo-1-octanol (1.1eq) in acetone (5ml) taken in screwcap vials (10ml capacity), K₂CO₃ (6eq) was added. The reaction mixtures were then capped and heated with orbital shaking (225 rpm) at 70°C in a digiblock orbital shaker for 36h. They were allowed to attain room temperature and filtered in parallel through syringe tubes fitted with cotton using the filtration manifold and washed with 5ml of acetone each. The filtrates were concentrated using the speedvac. The residues obtained were dissolved in EtOAc (8ml) each, washed with 1N. NaOH (2 x 2ml), water (2 x 2ml) and dried (Na₂SO₄). These were filtered in parallel through syringe tubes fitted with cotton using the filtration manifold. The filtrates were then evaporated using the speedvac to obtain the product alcohols (**B**) (70-97%yield).

### C. Synthesis of mesylates (C)

To the solutions of the alcohols (**B**) and Et₃N (2eq) in CH₂Cl₂ (6ml) at 0°C, MsCl (1.5eq) was added and kept at 0°C using an ice bath for 3h with occasional stirring. They were diluted with CH₂Cl₂ (5ml) and washed with 1N. HCl (3 x 2ml), water (1 x 1ml) and brine (1 x 1ml) and dried (Na₂SO₄). These were filtered in parallel through syringe tubes fitted with cotton using the filtration manifold. Evaporation of solvent from the filtrates using the speedvac afforded the mesylates (**C**) (90-100% yield).

### D. Synthesis of esters (D-I, D-II)

To the solutions of the mesylates (C) and the acid (2eq) in DMF (6ml) taken in screwcap vials (10ml capacity), K₂CO₃ (leq) was added. The reaction mixtures were then capped and heated with orbital shaking (225 rpm) at 55°C in a digiblock shaker for 24h. They were diluted with EtOAc (20ml) and quenched with sat. NH₄Cl (5ml). The organic layers were separated and the aqueous layers were extracted with 10 ml more of EtOAc. The combined organic extracts for each reaction were then washed with sat. NaHCO₃ (2 x 5ml) and brine (2 x 5ml) and dried (Na₂SO₄). These were filtered in parallel through syringe tubes fitted with cotton using the filtration manifold. Removal of solvent from the filtrates using a speedvac furnished the esters **(D-I, D-II)** (59-91%yield).

### E. Synthesis of the quaternary salts (E-I, E-II)

To the solution of the esters **(D-I, D-II)** in DME (6ml) in screwcap vials (10ml capacity), MeI (30 eq) was added. The reaction mixtures were capped and heated with orbital shaking (225 rpm) at 85°C in a digiblock shaker for 36h. Then they were allowed to attain room temperature and solvent was completely evaporated using a speedvac. The crude products obtained were purified in parallel chromatography over Si gel columns (5 x 1cm). The columns were first eluted with CH₂Cl₂ (20ml), EtOAc (20ml) and then with 10%MeOH in CH₂Cl₂ (30ml) to obtain the quarternary salts **(E-I, E-II)** (40-92% yield) .

### EXAMPLE 6: GENERAL PROCEDURE FOR CRYSTALLIZATION, DATA COLLECTION AND DETERMINAITON OF STRUCTURAL RELATIONSHIP BETWEEN NAD SYNTHETASE INHIBITOR COMPOUNDS AND THE NAD SYNTHETASE ENZYME.

**A. Crystallization.** Protein was expressed and purified as described in the literature. (Nessi, C., Albertini, A., Speranza, M..L. & Galizzi, A. *The out B gene of Bacillus subtilis codes for NAD*^{*+*} *synthetase*. **J. Biological Chemistry** 270, 6181-6185). Crystals were grown by vapor diffusion at 28° C from 21 - 23% polyethylene glycol (PEG) 400, 100 mM acetate buffer, pH 5.2, 50 mM MgCl₂, 2.5 mM β -mercapto ethanol. Inhibitors were dissolved in minimal volume of PEG 400 and then mixed with crystallization medium to final concentration of 5 - 10 mM in 23% v/v PEG 400. 10 µl of protein solution (16 mg/ml in crystallization buffer) were mixed with 10 µL of inhibitor in crystallization medium incubated at 28 °C. The crystals of NAD synthetase complexed with inhibitors obtained belonged to space group P21 as described previously in the literature. (Rizzi, M., Nessi, C., Matteve, A., Coda, A. & Galizzi, A. *Crystal structure of NH*_{*3*}*-dependent NAD*^{*+*} *synthetase from Bacillus subtilis.* **EMBO Journal** 15, 5125-5134 (1996)).
**B. Data collection.** Diffraction data for the different complexes of NAD synthetase with inhibitors were collected at ambient temperature or at 120° K with use of R-axisII and R-axisIV image plates and a rotating anode X-ray source, using Xstream Cryosystem device. Data were processed with DENZO and SCALEPACK as described. (Otwinowski, Z., & Minor, W. Processing of X-ray data collected in oscillation mode. in Carter C.W Jr. and Sweet M.M ( eds.), **Methods of Enzymology,** *v*. 76, 307-326, Academic Press, New York (1996)). All subsequent calculations were performed with CCP4 program suite. (CCP4. The SERC (UK) Collaborative Computing Project No. 4, *A suite of Programs for Protein Crystallography*, SERC Daresbury Laboratory, Warrington, UK, 1979.)
**C. Refinement.** All complexes ofNAD synthetase with inhibitors were isomorphous with the recently solved structure NAD synthetase complexed with AMP, PPi, ATP and Mg²⁺ (Rizzi, M., Nessi, C., Bolognesi, M., Coda, A. & Galizzi, A. *Crystallization of NAD*^{*+*} *synthetase from Bacillus subtilis*. **Proteins** 26, 236-238 (1996). The coordinates from this structure excluding ligands and water molecules were used as a starting model for the free enzyme at 2.0 A resolution. Rigid-body refinement followed by simulated annealing were carried out with X-PLOR (Brunger, A.T., **X-PLOR Version 3.1. A system for X-ray Crystallography and NMR** ( Yale Univ Press, New Haven, CT, 1992)) until convergence was reached using all reflections to 2.0 A resolution. The model of the free enzyme was subsequently used for phasing and refinement of the complexes of NAD synthetase with inhibitors. The procedure for refinement with X-PLOR of a particular model included first simulated annealing cycle and positional refinement of the protein. Inhibitors were manually built into (Fₒ-F_{c})α_{c} difference Fourier maps using QUANTA ( Molecular Simulations ) (Jones, T., Zou, J., Cowan, S. & Kjeldgaard, M. *Improved method for building protein models in electron density maps and the location of the errors in these models.* **Acta Crystallogr. A 47**, 110-119 (1991)) and O and refinement continued. A bulk solvent correction were then applied and ordered water molecules added following standard criteria.

### EXAMPLE 7: "ONE-AT-A-TIME" IN-VITRO SCREENING METHOD

The "one-at-a-time" *in vitro* bacterial NAD synthetase enzyme activity assay described below was used to test for relative activities of selected active molecules and synthetic dimers. The method was used to test selected NAD synthetase inhibitor compounds of the library herein, as well as commercially available compounds predicted to have bacterial NAD synthetase enzyme activity inhibitor capabilities.

A solution (1014 L) of 60 mM HEPPS pH 8.5 with 20 mM KCl was prepared containing the following species: 0.210 mM ATP, 0.152 mM NaAD, 4 mM MgCl₂, 10 mM NH₄Cl, 0.21 mg/mL ADH, and 1% ETOH. A stock solution of test inhibitors was then prepared by dissolving solid samples into 100% DMSO. 20 L of the test compound stock solution was then added to the mixture to give the final test compound concentrations listed. To start the enzyme assay, 16 L of a 65 g/mL NAD Synthetase solution were added, the mixture was mixed three times, and the absorbance at 340 nm was then monitored kinetically for 400 s using an Aviv 14DS UV-Vis spectrophotmeter. The initial kinetics trace from 30 to approximately 250 seconds after enzyme addition was then fitted to a straight line using linear regression, and this rate was then compared to that of a control containing no inhibitor, using the following formula to calculate % Inhibition: {(Vo-V)/Vo}*100%, where Vo is the rate of the reaction with no test compound present and V is the rate of the reaction with test the test compound added. Each compound was tested in triplicate, and the resulting values for % inhibition were averaged to give the listed value. IC₅₀ values were obtained for select compounds by assaying,six different concentrations of test compound, in triplicate, at concentrations between 0.0 and 2.0 mM, and plotting the resulting % inhibition values against the -LOG of the test compound dose to reveal the concentration at which 50% inhibition was observed.

### EXAMPLE 8: COMPARISON OF BACTERIAL NAD SYNTHETASE ACTIVITY IN DIFFERENT BACTERIA TYPES

To determine initially if a compound found active in the assays, Compound 864, was also an effective inhibitor of a variety of different bacteria, a standard antibiotic assay was performed. The results are summarized in Table 306. In this assay 250 µg of Compound 864 (25 µg/ml in DMSO) was spotted on 6 or 7 mm paper disks. Each disk was placed on separate 30 ml solid-medium plates layered with bacteria. Blood agar plates were used for *Streptococcus*, and minimal-glucose plates were used for the other microorganisms in Table 306. DMSO controls provided negative results.

**TABLE 306:**

| **INHIBITION OF GRAM +/- BACTERIA BY COMPOUND 864** | | | |
|---|---|---|---|
| BACTERIUM | STRAIN | GRAM + OR - | ZONE OF INHIBITION (mm) |
| *Escherichia coli* K-12 | MG1655 | - | 9.5 |
| | (CGSC#6300) | | |
| *Escherichia coli* K-12 | W3110 | - | 9.5 |
| | (CGSC#4474) | | |
| *Salmonella typhimurium* | LT2 TT366 | - | 10 |
| *Streptococcus pneumonia* | D39 | + | 12 |
| *Streptococcus pneumoniae* | WU2 | + | 15 |
| *Bacillus subtilis* | A700 | + | 19.5 |

Compound 864 demonstrates inhibitory activity from which bacterial NAD synthetase inhibitory activity in a variety of bacteria may be extrapolated. Further, it is evident from this data that inhibition of bacterial NAD synthetase enzyme corresponds to inhibition of both gram positive and gram negative bacteria. Such data also demonstrates the effectiveness of the compounds herein as bacteriacidal agents, antimicrobial agents and disinfectants.

### EXAMPLE 9: ADAPTATION OF ENZYME ASSAY TO HIGH THROUGH-PUT SCREENING OF INHIBITORS

The enzyme kinetics assay for bacterial NAD synthetase enzyme inhibitory activity utilized as the primary biological screen, discussed previously as the "one-at-a-time" *in vitro* assay, was adapted to a microtiter plate format so that many compounds could be screened in a short time *i.e.*, in a high-throughput system.

The final reaction mixture included 0.2ml of 60mM HEPPS buffer, pH 8.5, 10mM MgCl₂, 19mM NH₄Cl₂, 20mM KCl, 0.1mM NaAD, 0.3% n-Octyl--D-Glucopyranoside, 1% ethanol, 1g/ml NAD synthetase, 62.5g/ml yeast alcohol dehydrogenase, 0.2mM ATP and 2.5% DMSO.

The measurement of inhibitory activities of the test compounds was conducted using a high through-put screening system (HTS system). The HTS system utilizes an integrated Sagian 2M ORCA robotic system coordinating the functions of a Beckman Biomek 2000 liquid handler and a Molecular Devices SpectraMax Plus spectrophotometer. The 2M ORCA robotic station was responsible for the movement of all hardware and the integration of multiple stations on the worksurface. The Biomek 2000 is programmed to perform all phases of liquid dispensing and mixing. The SpectraMax Plus spectrophotometer was equipped to monitor absorbance in a 96- well plate format.

The present assay was designed for a 96-well plate format and begun with the dispensing of 0.170ml of reaction buffer containing 60mM HEPPS buffer, pH 8.5, 10mM MgCl₂, 19mM NH₄Cl₂, 20mM KCl, 0.118mM NaAD, 0.3% n-Octyl--D-Glucopyranoside, 1.18% ethanol, 1.18g/ml NAD synthetase, and 73.75g/ml yeast alcohol dehydrogenase. Once the Biomek 2000 has completed this stage of the liquid handling, a 0.005ml volume of test compound in 100% DMSO or a 0.005ml of DMSO was dispensed in the reaction well. The Biomek 2000 mixed these components utilizing a predefined mixing program. The reaction was initiated by the addition of 0.025ml of a solution of 1.6mM ATP dissolved in 60mM HEPPS buffer, pH 8.5, 10mM MgCl₂, 19mM NH₄Cl₂, 20mM KCl, 2.5% DMSO, and 0.3% n-Octyl--D-Glucopyranoside. The reactions were monitored by measuring the increase in absorbance at 304nm. The linear portion of the reaction was monitored for 180sec. The initial velocity was determined using Softmax Pro, the software supplied with the Molecular Devices SpectraMax Plus spectrophotometer.

The compounds were supplied as a stock with a concentration of 50mM dissolved in 100% DMSO. An initial screen was conducted on all compounds using a 2 or 3 concentration screen. The 2 panel screen used concentrations of 0.2mM and 0.1mM for the compounds. The 3 panel screen used concentrations of 0.2mM, 0.1mM, and 0.05mM. From the initial screen, lead compounds which indicated the greatest inhibitory capacity were then subjected to a wider screen of concentrations (0.1mM to 0.005mM) to determine the apparent IC-50 values for each compound.

Double reciprocal plots of initial velocities have yielded the kinetic parameters given in the following table for the 2 mL cuvette assay. Also included in the table are the Km values obtained in the 0.2 mL microtiter plate assay. In this latter assay, a Beckmann/Sagian automated robotic system was applied in for high through-put screening in one preferred embodiment of the method.

**TABLE 308:**

| **KINETIC DATA FOR HIGH THROUGH-PUT SCREENING METHOD** | | | |
|---|---|---|---|
| **2 mL Assay** | | | **0.2 mL Assay** |
| **Substrate** | **Km (mM)** | **Vmax (nM/sec)** | **Km (mM)** |
| **Mg**^{**+2**} | 2.6 | 120 | 2.9 |
| **NH**_{**3**} | 2.88 | 137 | -- |
| **ATP** | 0.12 | 436 | 0.152 |
| **NaAD** | 0.075 | 286 | 0.076 |

With the preferred high through-put system and the adapted enzymatic screening assay for bacterial NAD synthetase inhibitory enzyme activity described previously, large numbers of compounds can be screened in a short period.

### EXAMPLE 10: NAD SYNTHETASE INHIBITORY ACTIVITY OF COMPOUNDS

Compounds of the libraries herein were screened using the high through-put enzyme kinetics assays described above in Example 9. Table 312B, below presents NAD synthetase enzyme inhibition data for a number of compounds of the libraries herein tested at 0.2 mM, mM dose,

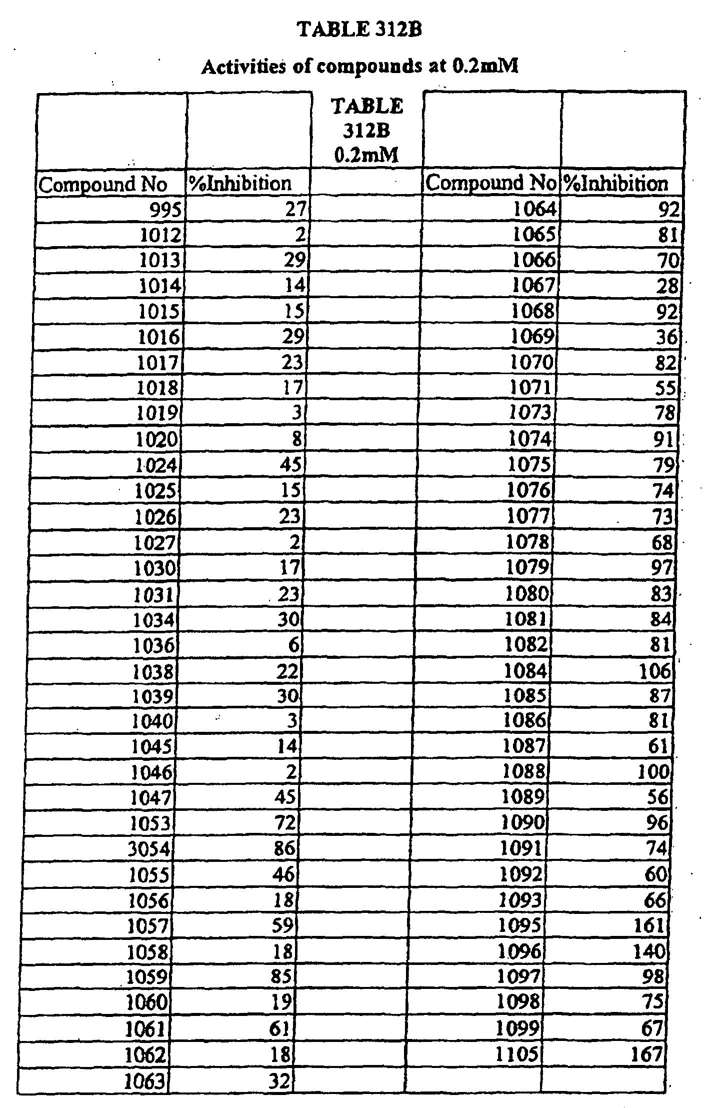

Tables 318B sets out IC₅₀ values for compounds of the invention herein. Table 318B sets out values for a number of compounds of the invention herein, some of which are also considered to be potent compounds. The potency of the compounds is expressed according to IC₅₀ values. The IC₅₀ value is that amount of NAD synthetase enzyme inhibitor compound required to inhibit the enzyme by 50%.

**TABLE 318B**

| **IC**_{**50**} **DATA FOR SELECTED COMPOUNDS** | | |
|---|---|---|
| | **TABLE 318B** | |
| **COMPOUND NUMBER** | | **IC**_{**50**} **of Compound s(µM)** |
| 1031 | | 36.3 |
| 1064 | | 37.5 |
| 1065 | | 37.5 |
| 1068 | | 62.5 |
| 1070 | | 36.2 |
| 1074 | | 30 |
| 1075 | | 65 |
| 1076 | | 135 |
| 1078 | | 122.5 |
| 1079 | | 36.2 |
| 1080 | | 100 |
| 1081 | | 67.5 |
| 1082 | | 50 |
| 1084 | | 12.5 |
| 1085 | | 137.5 |
| 1086 | | 27.5 |
| 1087 | | 40 |
| 1090 | | 21.2 |
| 1095 | | 100 |
| 1096 | | 68.9 |
| 1097 | | 63.7 |
| 1099 | | 100 |
| 1104 | | 61 |

Other embodiments of the invention will be apparent to those skilled in the art from consideration of the specification and practice of the invention disclosed herein. It is intended that the specification and examples be considered as exemplary only, with the true scope and spirit of the invention being indicated by the following claims.

## Claims

1. A compound of the formula: 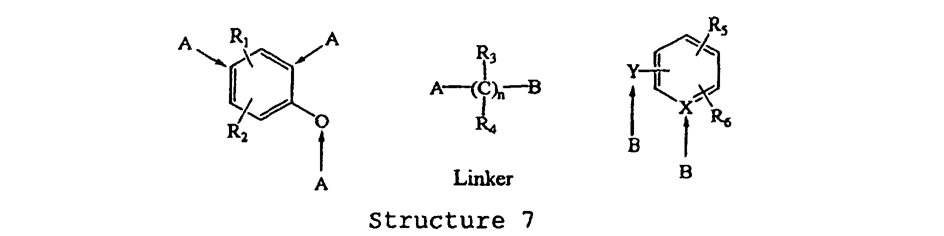 wherein X is C, N, O, or S, Y is C, N, O, S, carboxy, ester, amide, or ketone, A and B represent the respective sites of attachment for a linker,
n is an integer of from 1 to 12, and
R₁-R₆ each, independently, is H,
aryl, hydroxy, ketone, nitro, amino, amidino, guanidino, carboxylate, amide, sulfonate, halogen, a substituted or unsubstituted branched, un-branched or cyclic aliphatic group,
the linker is a branched, un-branched or cyclic aliphatic group, wherein aliphatic groups include alkyl, alkenyl and alkynyl groups,
the linker can further include heteroatoms, when R₁-R₆ represents a cyclic group, the group can include one or more closed rings, and
the compound inhibits the NAD synthetase enzyme of a microbe.

2. The compound of claim 1, wherein n is an integer of from 3 to 10.

3. The compound of claim 1 or 2, wherein n is an integer of from 5 to 9.

4. The compound of any of claims 1-3, wherein n is an integer of from 6 to 9.

5. The compound of any of claims 1-4, wherein R₁-R₆ each, independently, is H, alkyl, alkenyl, alkynyl, or aryl.

6. A compound of the formula: 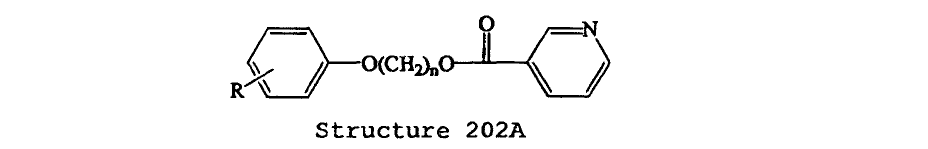 wherein n is an integer of from 1 to 12 and wherein R is H; 4-NO₂; 2-CONHPh; 2-NO₂; 4 [1' (4'-acetylpiperazine)]; 2-COCH₃; 3-OCOCH₃; 3-OCH₃; 4-COCH₃; 3-OCOPh: 2-CONH₂; 4-CH=CHCOCH₃; 4-OCOPh; 4-CH=CHCOPh; 4-{CO-3'[2'-butylbenzo(*b*)furan]}; 3-NO₂; 4-[5'-(5'-phenylhydantoin)]; 2-CH=CHCOPh; 2-OCH₃; 4-COPh; 4-CONH₂; 3-COCH₃; 4-OPh; 4-(N-phthalimide); 3-(N-morpholine); 2-(N-pyrrolidine); 2-(N-morpholine); or 4-OCH₂Ph.

7. A compound of the formula: 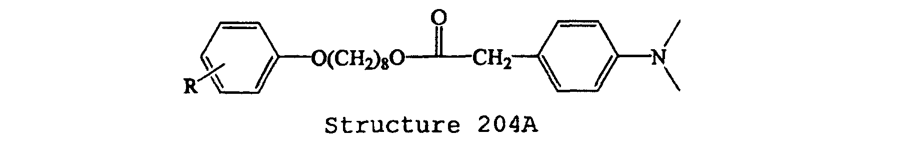 wherein n is an integer of from 1 to 12 and wherein R is H; 4-NO₂; 2-CONHPh; 2-NO₂; 4[1'(4'-acetylpiperazine)]; 2-COCH₃; 3-OCOCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-butylbenzo(*b*)furan]}; 3-NO₂; 4-[5'-(5'-phenylhydantoin)]; 2-CH=CHCOPh; 2-OCH₃; 4-COPh; 4-CONH₂; 3-COCH₃; 4-OPh; 4-(N-phthalimide); 3-(N-morpholine); 2-(N-pyrrolidine); 2-(N-morpholine); or 4-OCH₂Ph.

8. A compound of the formula: 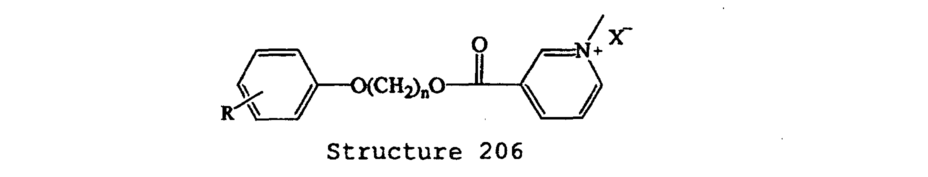 wherein n is an integer of from 1 to 12 and R is H; 4-NO₂; 2-CONHPh; 2-NO₂; 2-COCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-butylbenzo(*b*)furan]}; 3-NO₂; 2-CH=CHCOPh; 2-OCH₃; 4-COPh; 3-COCH₃; 4-OPh; 4-(N-phthalimide); or 4-OCH₂Ph; wherein X- is an anion.

9. A compound of the formula: 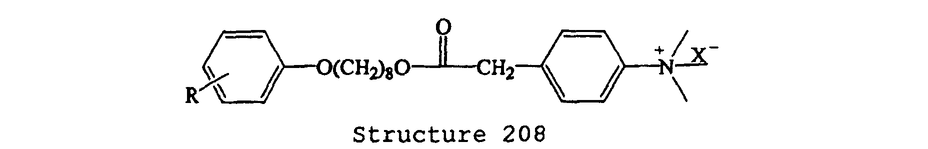 wherein R is 4-NO₂; 2-CONHPh; 2-NO₂; 2-COCH₃; 3-OCH_{3;} 4-COCH₃; 3-OCOPh; 2-CONH₂; 4-CH=CHCOCH₃; 4-OCOPh; 4-CH=CHCOPH; 4-{CO-3'[2'butylbenzo(*b*)furan]}; 3-NO₂; 2-CH=CHCOPh: 2-OCH₃; 4-COPh; 3-COCH₃; 4-OPh; 4-(N-phthalimide); 3-(N-morpholine); 2-(N-morpholine); or 4-OCH₂Ph; wherein X- is an anion.

10. A compound of the formula: 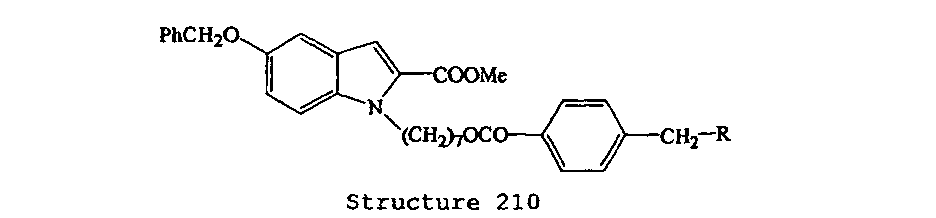 wherein R is NH₂; NMe₂; NMe₃.I; NH₂.HCl; or NMe₂.HCl.

11. A compound of the formula: 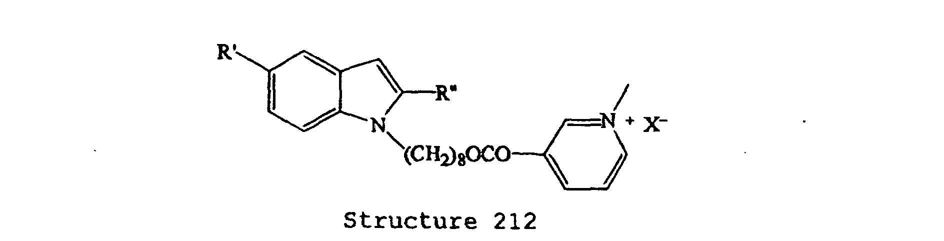 wherein R' is PhCONH or Ph₃C and R" is H or COOCH₃;
wherein X- is an anion.

12. A compound of the formula: 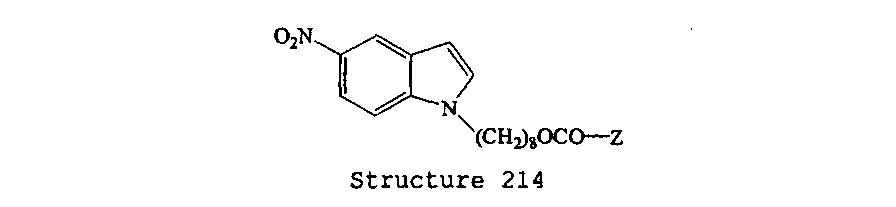 wherein Z is 4-hydroxyphenyl or 3-hydroxy-4-methylphenyl.

13. A compound of the formula, wherein X- is an anion: 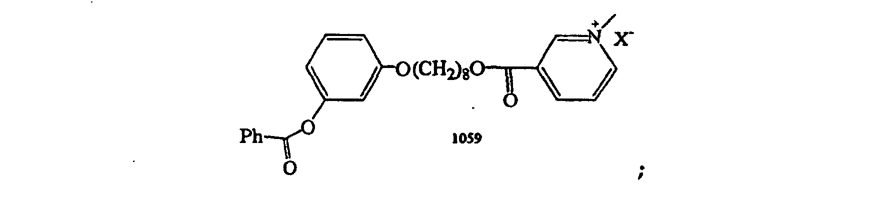 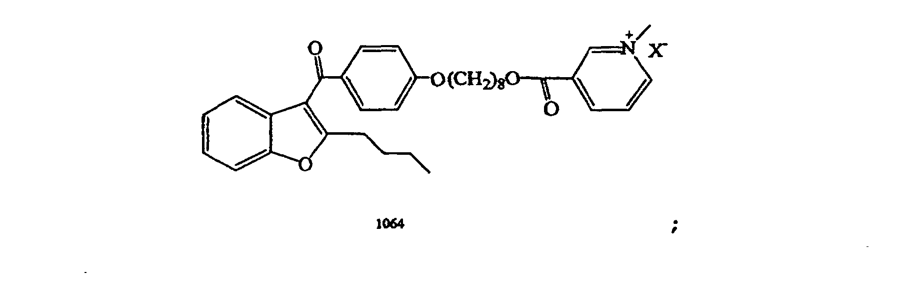 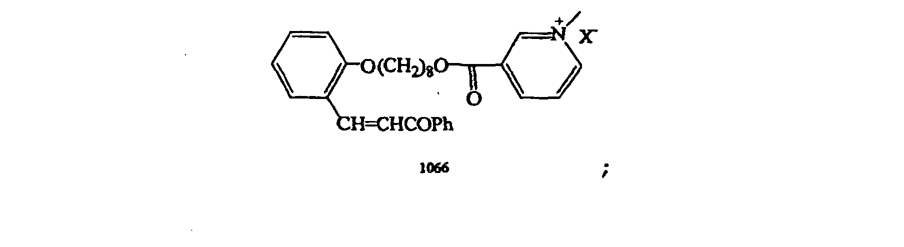 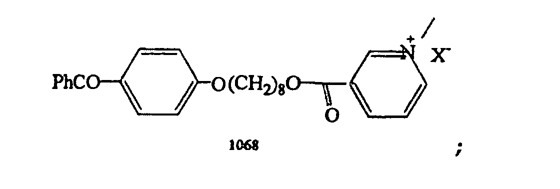 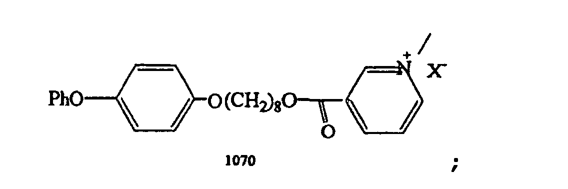 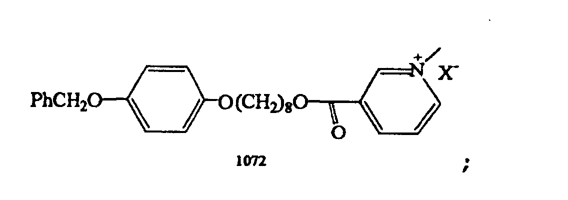 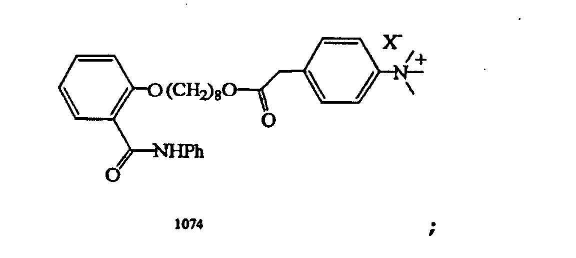 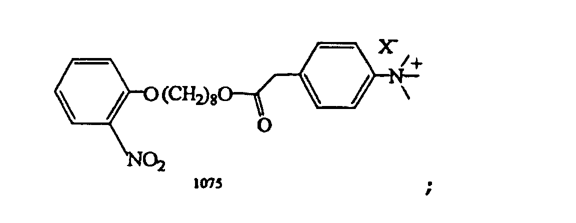 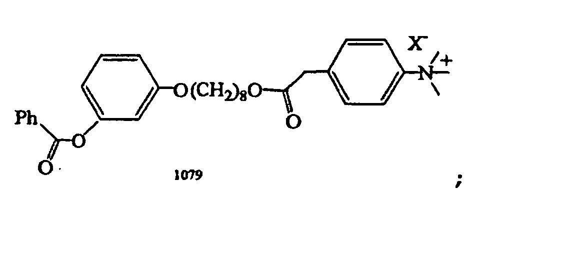 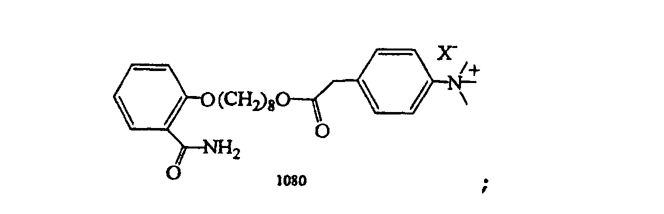 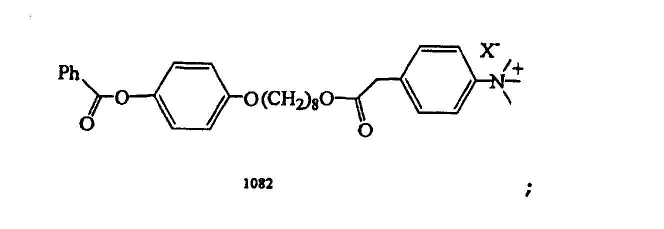 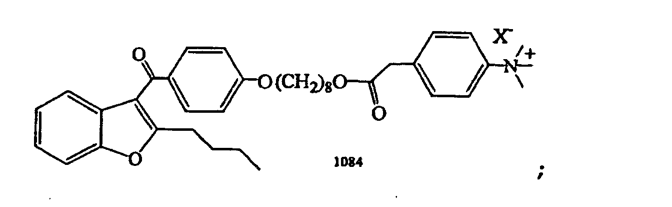 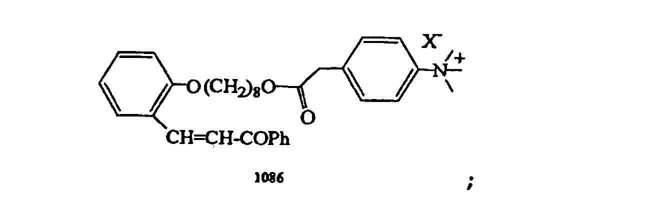 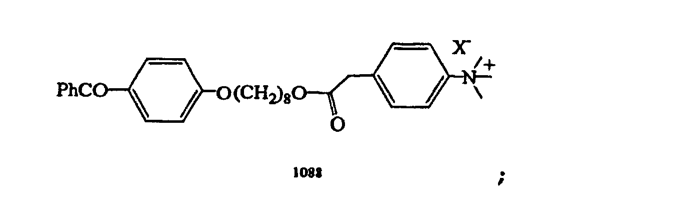 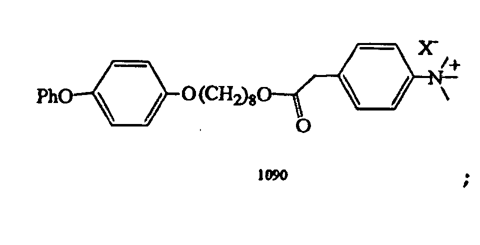 or

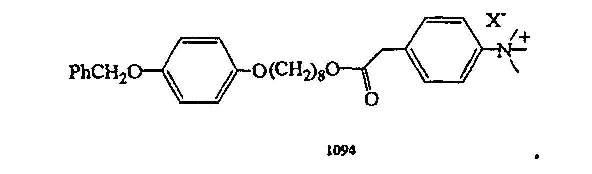

14. The compound of claim 10, which has the formula:

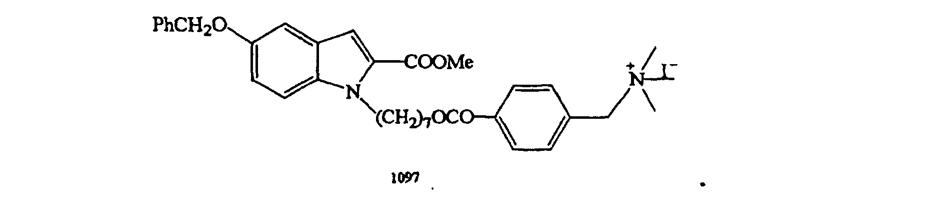

15. A compound of the formula: 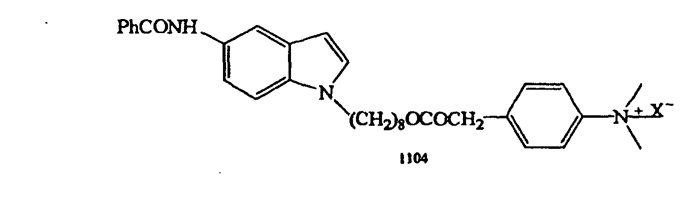 or 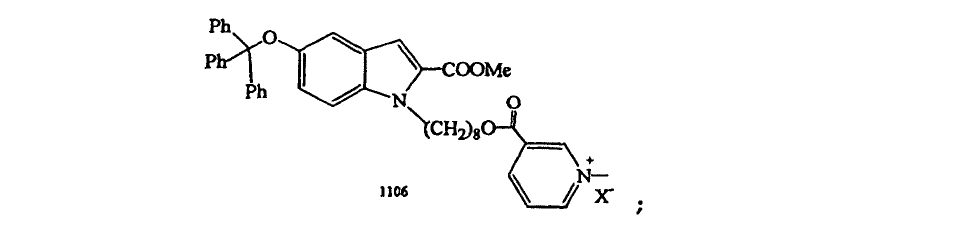 wherein X- is an anion.

16. The compound of claim 13, which has the formula:

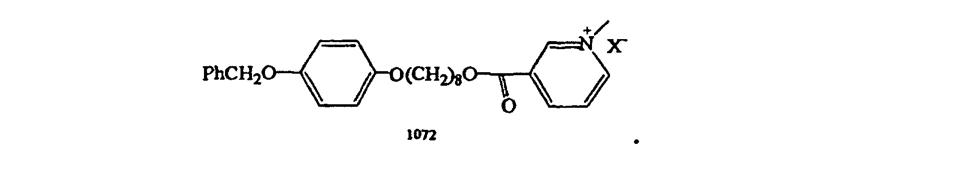

17. The compound of claim 13, which has the formula:

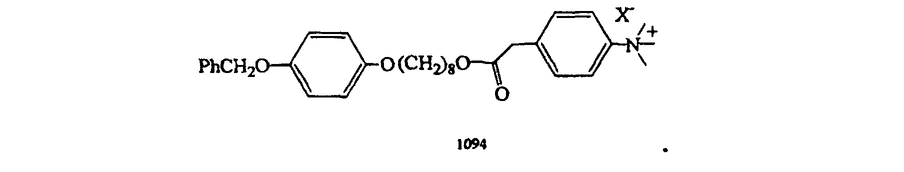

18. The compound of any of claims 10-17, which inhibits the NAD synthetase enzyme of a microbe.

19. The compound of any of claims 1-18, which inhibits the growth of the microbe with an IC₅₀ of about 100 µM or less.

20. The compound of any of claims 1-19, which binds to at least one receptor site of the NAD synthetase enzyme of a microbe.

21. A formulation which comprises as an active ingredient a compound as claimed in any of claims 1-20 associated with one or more acceptable carriers, medicinal agents, pharmaceutical agents, adjuvants, or diluents.

22. The compound of any of claims 1-20 for use in medicine.

23. Use of the compound of any of claims 1-20 in the manufacture of a medicament for the treatment or prevention of a bacterial infection.

24. Pharmaceutical for the prevention or treatment of bacterial infection **characterized in that** it contains a compound of any of claims 1-20 as an active substance.

25. A method of making a bacterial NAD synthetase inhibitor compound comprising:
(a) brominating an aniline with N-bromosuccinimide to form a 2-bromo-R¹ substituted-aniline or a 2 -bromo-R²-substituted-aniline; (b) reacting the 2-bromo-R¹-substituted-aniline or a 2-bromo-R²-substituted-aniline using a Heck coupling reaction to form an alkyne-substituted aniline; (c) reacting the alkyne-substituted aniline using a cyclization reaction to form an indole alcohol; (d) quaternizing the indole alcohol with an amine; (e) reacting the indole alcohol with triflouromethylsulfonic anhydride to provide an indole triflate; and (f) reacting the indole triflate with an amine to form an indole alkylammonium product.

26. A method of making a bacterial NAD synthetase inhibitor compound comprising:
(a) alkylating a phenol with 7-bromo-1-heptanol to provide 7-(phenyloxy)-1-heptanol; (b) mesylating 7-(phenyloxy)-1-heptanol to provide 7-(phenyloxy)-1-heptyl methanesulfonate; (c) esterifying 7-(phenyloxy)-1-heptyl-methanesulfonate to provide 7-(phenyloxy)-1-heptyl nicotinate; and (d) N-methylating 7-(phenyloxy)-1-heptyl nicotinate to provide [7-(phenyloxy)-1-heptyl-(N-methyl) nicotinate] iodide.

27. A disinfecting composition comprising a compound of any of claims 1-20.

## Patentansprüche

1. Verbindung der Formel: 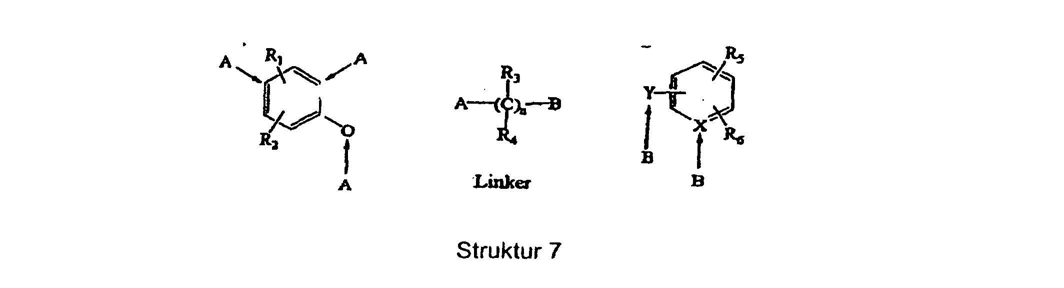 worin X C, N, O oder S ist, Y C, N, O, S, Carboxy, Ester, Amid oder Keton ist, A und B die jeweiligen Verknüpfungsstellen für einen Linker bedeuten,
n eine ganze Zahl von 1 bis 12 ist, und
R₁-R₆ jeweils unabhängig H, Aryl, Hydroxy, Keton, Nitro, Amino, Amidino, Guanidino, Carboxylat, Amid, Sulfonat, Halogen, eine substituierte oder unsubstituierte verzweigte, unverzweigte oder cyclische aliphatische Gruppe ist,
der Linker eine verzweigte, unverzweigte oder cyclische aliphatische Gruppe ist, wobei zu aliphatischen Gruppen Alkyl-, Alkenyl- und Alkinylgruppen gehören,
der Linker außerdem Heteroatome enthalten kann,
wenn R₁-R₆ eine cyclische Gruppe bedeutet, die Gruppe einen oder mehrere geschlossene Ringe enthalten kann,und
die Verbindung das NAD-Synthetase-Enzym eines Mikroorganismus hemmt.

2. Verbindung nach Anspruch 1, worin n eine ganze Zahl von 3 bis 10 ist.

3. Verbindung nach Anspruch 1 oder 2, worin n eine ganze Zahl von 5 bis 9 ist.

4. Verbindung nach einem der Ansprüche 1-3, worin n eine ganze Zahl von 6 bis 9 ist.

5. Verbindung nach einem der Ansprüche 1-4, worin R₁-R₆ jeweils unabhängig H, Alkyl, Alkenyl, Alkinyl oder Aryl ist.

6. Verbindung der Formel: 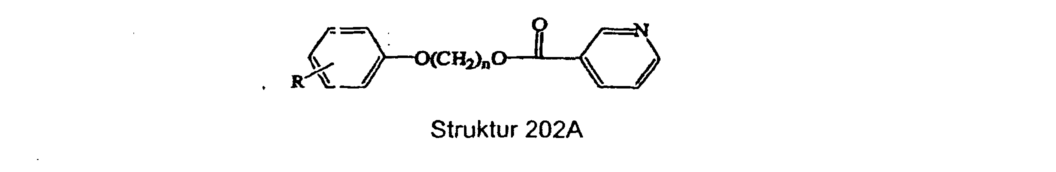 worin n eine ganze Zahl von 1 bis 12 ist und worin R H; 4-NO₂; 2-CONHPh; 2-NO₂; 4[1'(4'-Acetylpiperazin)]; 2-COCH₃; 3-OCOCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-Butylbenzo(*b*)furan]}; 3-NO₂; 4-[5'-(5'-Phenylhydantoin)]; 2-CH=CHCOPh; 2-OCH₃; 4-COPh; 4-CONH₂; 3-COCH₃; 4-OPh; 4-(N-Phthalimid); 3-(N-Morpholin); 2-(N-Pyrrolidin); 2-(N-Morpholin); oder 4-OCH₂Ph ist.

7. Verbindung der Formel: 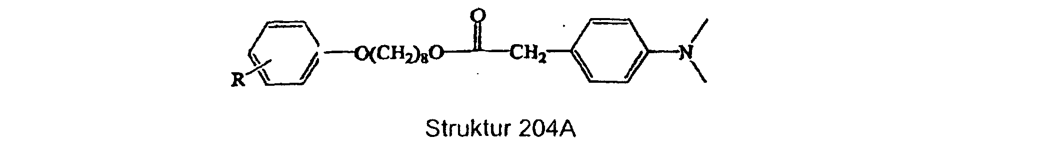 worin n eine ganze Zahl von 1 bis 12 ist und worin R H; 4-NO₂; 2-CONHPh; 2-NO₂; 4[1'(4'-Acetylpiperazin)]; 2-COCH₃; 3-OCOCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-Butylbenzo(*b*)furan]}; 3-NO₂; 4-[5'-(5'-Phenylhydantoin)]; 2-CH=CHCOPh; 2-OCH₃; 4-COPh; 4-CONH₂; 3-COCH₃; 4-OPh; 4-(N-Phthalimid); 3-(N-Morpholin); 2-(N-Pyrrolidin); 2-(N-Morpholin); oder 4-OCH₂Ph ist.

8. Verbindung der Formel: 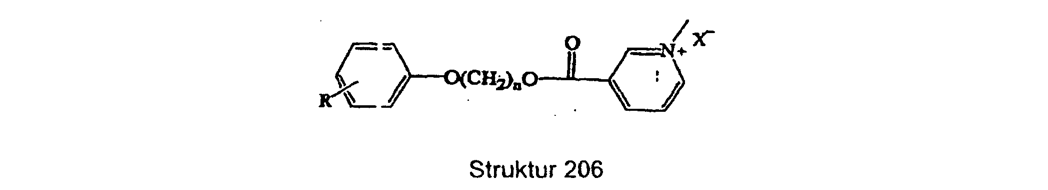 worin n eine ganze Zahl von 1 bis 12 ist und R H; 4-NO₂; 2-CONHPh; 2-NO₂; 2-COCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-Butylbenzo(*b*)furan]}; 3-NO₂; 2-CH=CHCOPh; 2-OCH₃; 4-COPh; 3-COCH₃; 4-OPh; 4-(N-Phthalimid); oder 4-OCH₂Ph ist; worin X- ein Anion ist.

9. Verbindung der Formel: 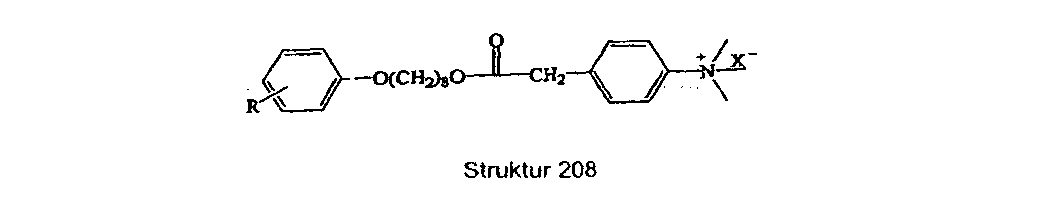 worin R 4-NO₂; 2-CONHPh; 2-NO₂; 2-COCH₃; 3-OCH₃; 4-COCH₃; 3-OCOPh; 2-CONH₂; 4-CH=CHCOCH₃; 4-OCOPh; 4-CH=CHCOPh; 4-{CO-3'[2'-Butylbenzo-(*b*)furan]}; 3-NO₂; 2-CH=CHCOPh; 2-OCH₃; 4-COPh; 3-COCH₃; 4-OPh; 4-(N-Phthalimid); 3-(N-Morpholin); 2-(N-Morpholin); oder 4-OCH₂Ph ist; worin X-ein Anion ist.

10. Verbindung der Formel: 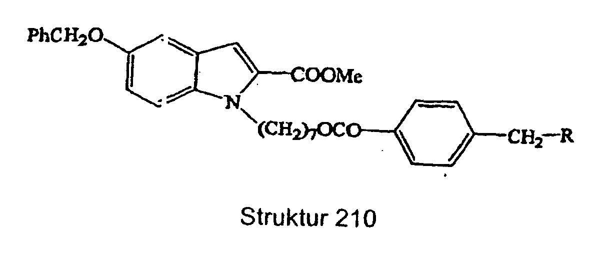 worin R NH₂; NMe₂; NMe₃•I, NH₂•HCl; oder NMe₂•HCl ist.

11. Verbindung der Formel: 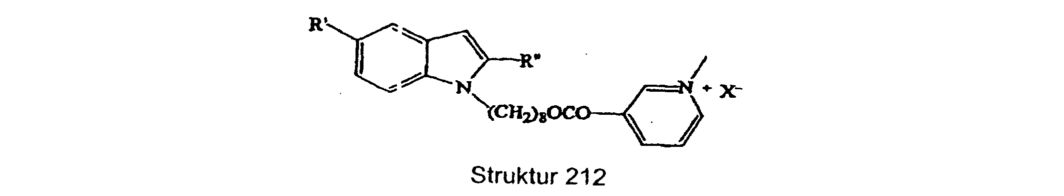 worin R' PhCONH oder Ph₃C ist und R" H oder COOCH₃ ist; worin X- ein Anion ist.

12. Verbindung der Formel: 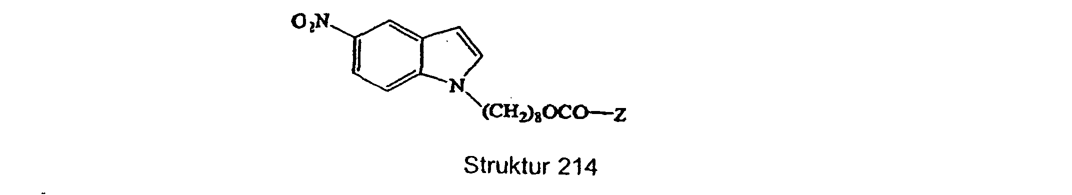 worin Z 4-Hydroxyphenyl oder 3-Hydroxy-4-methylphenyl ist.

13. Verbindung der Formel, worin X- ein Anion ist: 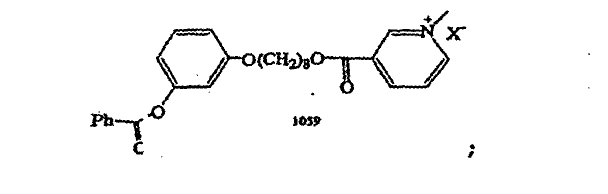 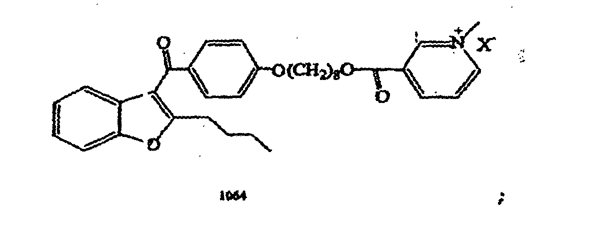 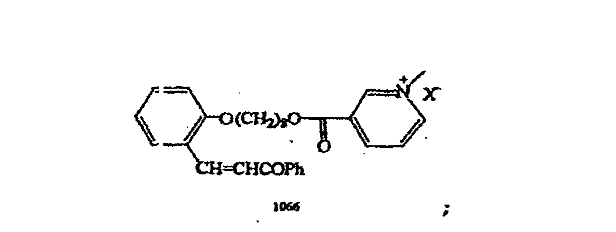 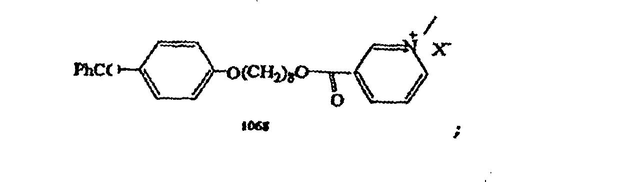 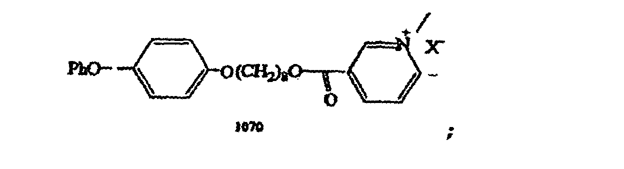 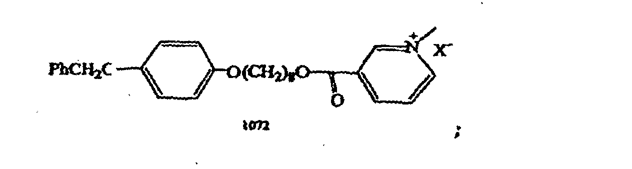 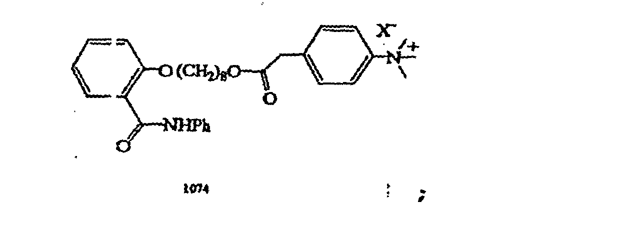 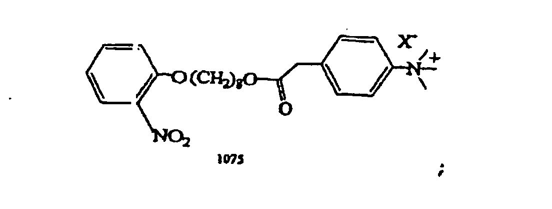 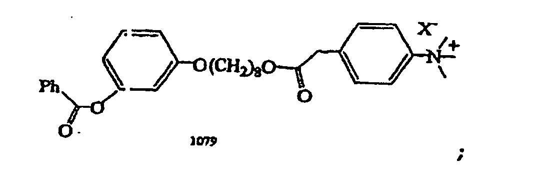 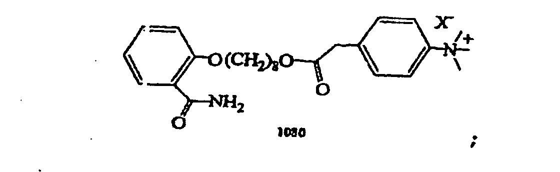 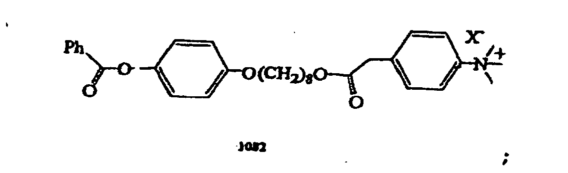 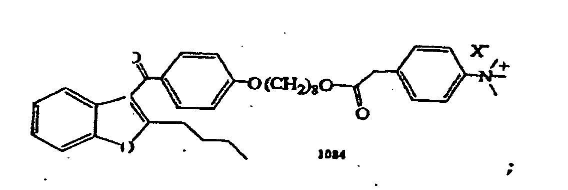 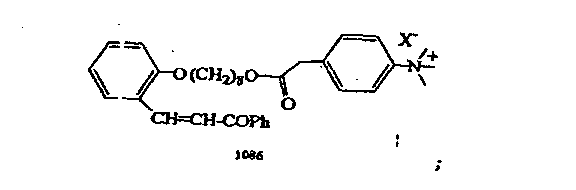 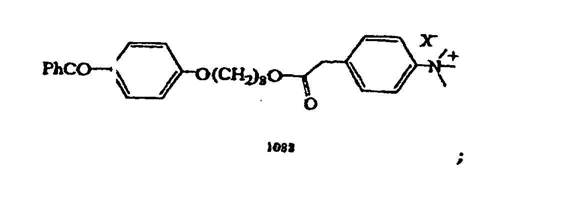 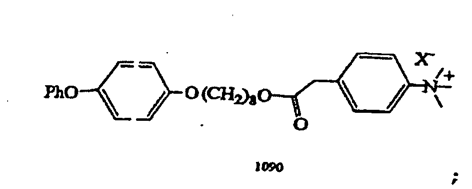 oder

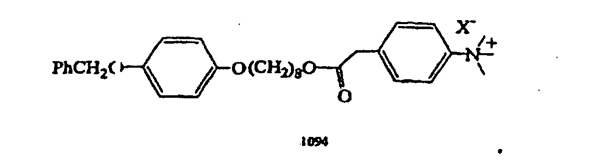

14. Verbindung nach Anspruch 10, welche die Formel aufweist:

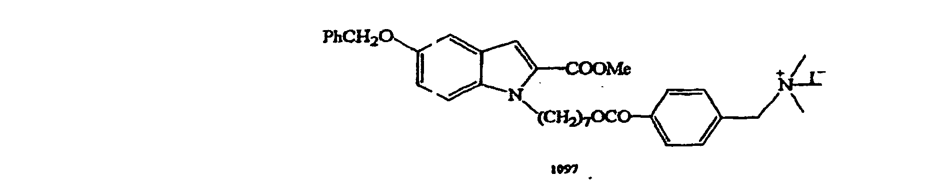

15. Verbindung der Formel: 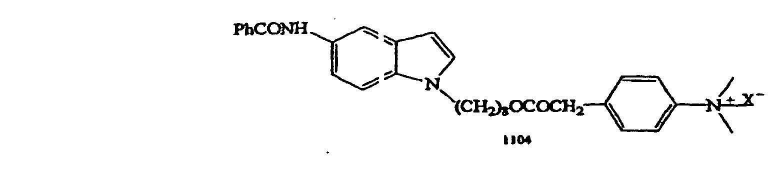 oder 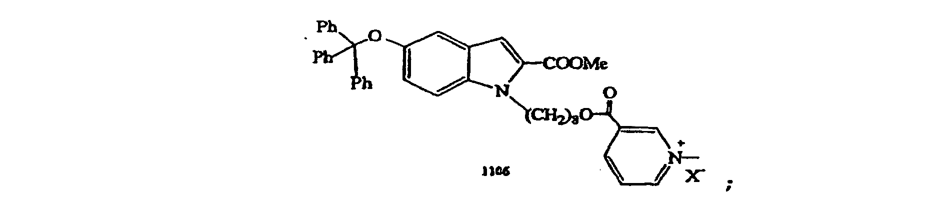 worin X⁻ ein Anion ist.

16. Verbindung nach Anspruch 13, welche die Formel aufweist:

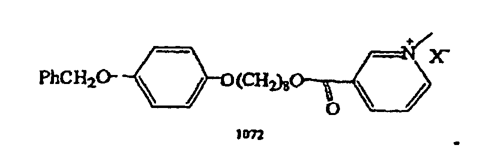

17. Verbindung nach Anspruch 13, welche die Formel aufweist:

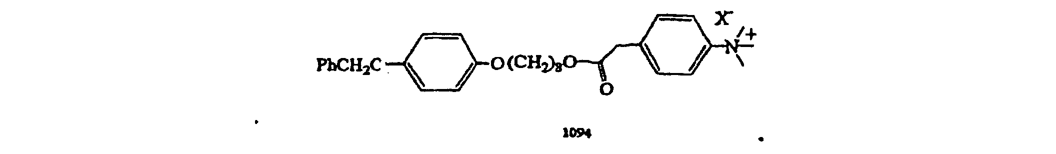

18. Verbindung nach einem der Ansprüche 10-17, welche das NAD-Synthetase-Enzym eines Mikroorganismus hemmt.

19. Verbindung nach einem der Ansprüche 1-18, welche das Wachstum des Mikroorganismus mit einer IC₅₀ von ungefähr 100 µM oder weniger hemmt.

20. Verbindung nach einem der Ansprüche 1-19, welche an wenigstens eine Rezeptorstelle des NAD-Synthetase-Enzyms eines Mikroorganismus bindet.

21. Formulierung, welche als Wirkstoff eine Verbindung nach einem der Ansprüche 1-20 in Verbindung mit einem oder mehreren annehmbaren Trägern, medizinischen Mitteln, pharmazeutischen Mitteln, Adjuvantien oder Verdünnungsmitteln umfasst.

22. Verbindung nach einem der Ansprüche 1-20 zur Verwendung in der Medizin.

23. Verwendung der Verbindung nach einem der Ansprüche 1-20 bei der Herstellung eines Medikaments für die Behandlung oder Verhütung einer Bakterieninfektion.

24. Pharmazeutikum für die Verhütung oder Behandlung einer Bakterieninfektion, **dadurch gekennzeichnet, dass** es eine Verbindung nach einem der Ansprüche 1-20 als Wirksubstanz enthält.

25. Verfahren zum Herstellen einer Bakterien-NAD-Synthetase-Inhibitorverbindung, umfassend:
(a) Bromieren eines Anilins mit N-Bromsuccinimid, um ein 2-Brom-R¹-substituiertes Anilin oder ein 2-Brom-R²-substituiertes Anilin zu bilden; (b) Umsetzen des 2-Brom-R¹-substituierten Anilins oder eines 2-Brom-R²-substituierten Anilins unter Verwendung einer Heck-Kupplungsreaktion, um ein Alkin-substituiertes Anilin zu bilden; (c) Umsetzen des Alkin-substituierten Anilins unter Verwendung einer Cyclisierungsreaktion, um einen Indolalkohol zu bilden; (d) Quaternisieren des Indolalkohols mit einem Amin; (e) Umsetzen des Indolalkohols mit Trifluormethylsulfonsäureanhydrid, um ein Indol-triflat bereitzustellen; und (f) Umsetzen des Indol-triflats mit einem Amin, um ein Indolalkylammoniumprodukt zu bilden.

26. Verfahren zum Herstellen einer Bakterien-NAD-Synthetase-Inhibitorverbindung, umfassend:
(a) Alkylieren eines Phenols mit 7-Brom-1-heptanol, um 7-(Phenyloxy)-1-heptanol bereitzustellen; (b) Mesylieren von 7-(Phenyloxy)-1-heptanol, um 7-(Phenyloxy)-1-heptyl-methansulfonat bereitzustellen; (c) Verestern von 7-(Phenyloxy)-1-heptyl-methansulfonat, um 7-(Phenyloxy)-1-heptyl-nicotinat bereitzustellen; und (d) N-Methylieren von 7-(Phenyloxy)-1-heptyl-nicotinat, um [7-(Phenyloxy)-1-heptyl-(N-methyl)-nicotinat]iodid bereitzustellen.

27. Desinfektionszusammensetzung, umfassend eine Verbindung nach einem der Ansprüche 1-20.

## Revendications

1. Composé de formule : 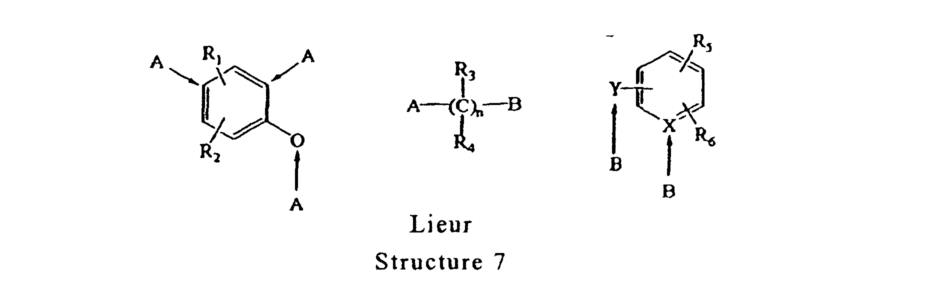 dans laquelle X est C, N, O ou S, Y est C, N, O, S, carboxy, ester, amide ou cétone, A et B représentent les sites respectifs d'attachement pour un lieur,
n est un entier de 1 à 12, et
R₁ à R₆ sont chacun, indépendamment, un atome d'hydrogène, un groupe aryle, hydroxy, cétone, nitro, amino, amidino, guanidino, carboxylate, amide, sulfonate, un atome d'halogène, un groupe aliphatique substitué ou non substitué, ramifié, non ramifié ou cyclique,
le lieur est un groupe aliphatique ramifié, non ramifié ou cyclique, où les groupes aliphatiques comprennent des groupes alkyle, alcényle et alcynyle,
le lieur peut de plus comprendre des hétéroatomes,
lorsque R₁ à R₆ représentent un groupe cyclique, le groupe peut comprendre un ou plusieurs cycles fermés, et
le composé inhibe l'enzyme NAD synthétase d'un microbe.

2. Composé selon la revendication 1, dans lequel n est un entier de 3 à 10.

3. Composé selon les revendications 1 ou 2, dans lequel n est un entier de 5 à 9.

4. Composé selon l'une quelconque des revendications 1 à 3, dans lequel n est un entier de 6 à 9.

5. Composé selon l'une quelconque des revendications 1 à 4, dans lequel R₁ à R₆ sont chacun indépendamment, un atome d'hydrogène, un groupe alkyle, alcényle, alcynyle ou aryle.

6. Composé de formule : 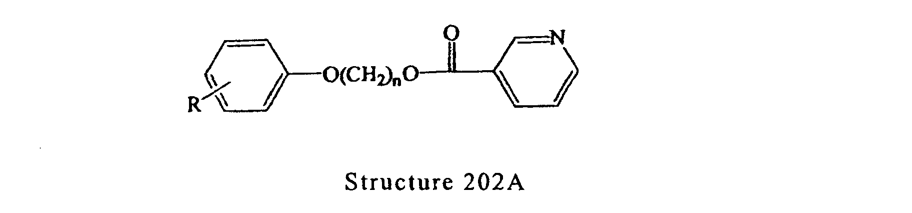 dans laquelle n est un entier de 1 à 12 et dans laquelle R est un atome d'hydrogène ; un groupe 4-NO₂ ; 2-CONHPh ; 2-NO₂ ; 4-[1'-(4'-acétylpipérazine)] ; 2-COCH₃ ; 3-OCOCH₃ ; 3-OCH₃ ; 4-COCH₃ ; 3-OCOPh ; 2-CONH₂ ; 4-CH=CHCOCH₃ ; 4-OCOPh ; 4-CH=CHCOPh ; 4-{CO-3'-[2'-butylbenzo(b)furane]} ; 3-NO₂ ; 4-[5'-(5'-phénylhydantoïne)] ; 2-CH=CHCOPh ; 2-OCH₃ ; 4-COPh ; 4-CONH₂ ; 3-COCH₃ ; 4-OPh ; 4-(N-phtalimide) ; 3-(N-morpholine) ; 2-(N-pyrrolidine) ; 2-(N-morpholine) ; ou 4-OCH₂Ph.

7. Composé de formule : 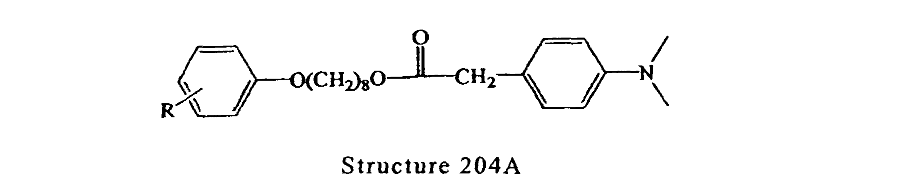 dans laquelle n est un entier de 1 à 12 et dans laquelle R est un atome d'hydrogène ; un groupe 4-NO₂; 2-CONHPh ; 2-NO₂ ; 4-[1'-(4'-acétylpipérazine)] ; 2-COCH₃ ; 3-OCOCH₃ ; 3-OCH₃ ; 4-COCH₃ ; 3-OCOPh ; 2-CONH₂ ; 4-CH=CHCOCH₃ ; 4-OCOPh ; 4-CH=CHCOPh ; 4-{CO-3'-[2'-butylbenzo(b)furane]} ; 3-NO₂ ; 4-[5'-(5'-phénylhydantoïne)] ; 2-CH=CHCOPh ; 2-OCH₃ ; 4-COPh ; 4-CONH₂ ; 3-COCH₃ ; 4-OPh ; 4-(N-phtalimide) ; 3-(N-morpholine) ; 2-(N-pyrrolidine) ; 2-(N-morpholine) ; ou 4-OCH₂Ph.

8. Composé de formule : 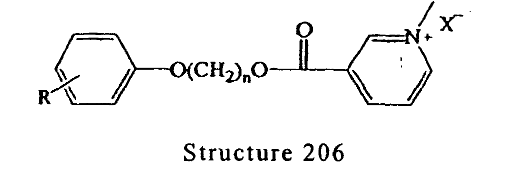 dans laquelle n est un entier de 1 à 12 et R est un atome d'hydrogène ; un groupe 4-NO₂ ; 2-CONHPh ; 2-NO₂ ; 2-COCH₃ ; 3-OCH₃ ; 4-COCH₃ ; 3-OCOPh ; 2-CONH₂ ; 4-CH=CHCOCH₃ ; 4-OCOPh ; 4-CH=CHCOPh ; 4-{CO-3'-[2'-butylbenzo(b)furane]} ; 3-NO₂ ; 2-CH=CHCOPh ; 2-OCH₃ ; 4-COPh ; 3-COCH₃ ; 4-OPh ; 4-(N-phtalimide) ; ou 4-OCH₂Ph ; dans laquelle X⁻ est un anion.

9. Composé de formule : 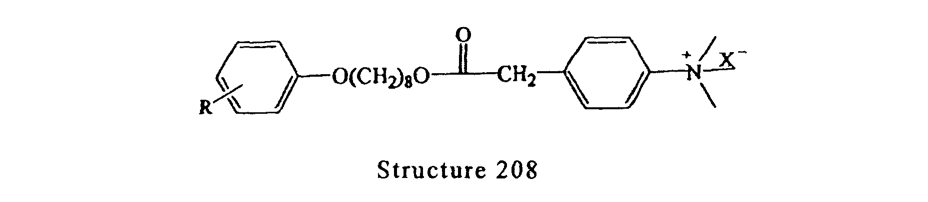 dans laquelle R est un groupe 4-NO₂ ; 2-CONHPh ; 2-NO₂ ; 2-COCH₃ ; 3-OCH₃ ; 4-COCH₃ ; 3-OCOPh ; 2-CONH₂ ; 4-CH=CHCOCH₃ ; 4-OCOPh ; 4-CH=CHCOPh ; 4-{CO-3'-[2'-butylbenzo(b)furane]} ; 3-NO₂ ; 2-CH=CHCOPh ; 2-OCH₃ ; 4-COPh ; 3-COCH₃ ; 4-OPh ; 4-(N-phtalimide) ; 3-(N-morpholine) ; 2-(N-morpholine) ; ou 4-OCH₂Ph ; dans laquelle X⁻ est un anion.

10. Composé de formule : 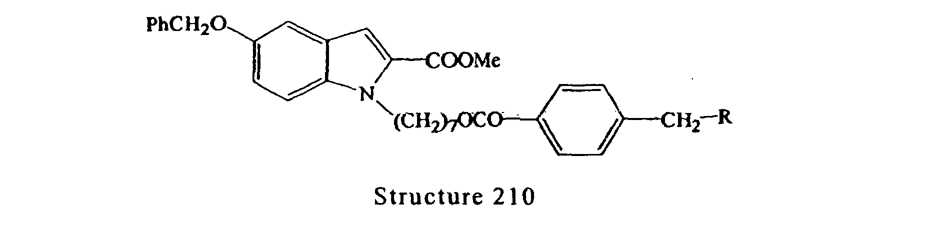 dans laquelle R est NH₂ ; NMe₂ ; NMe₃.I ; NH₂•HCl ou NMe₂•HCl.

11. Composé de formule : 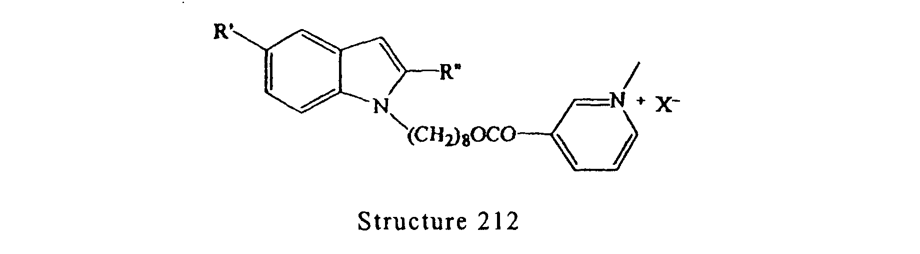 dans laquelle R' est PhCONH ou Ph₃C et R" est H ou COOCH₃ ;
dans laquelle X⁻ est un anion.

12. Composé de formule : 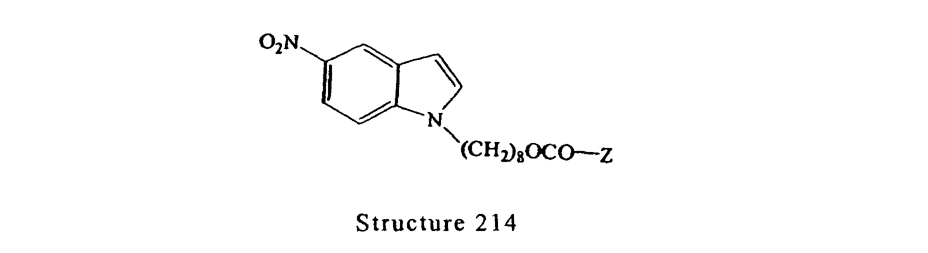 dans laquelle Z est un groupe 4-hydroxyphényle ou 3-hydroxy-4-méthylphényle.

13. Composé de formule, où X⁻ est un anion : 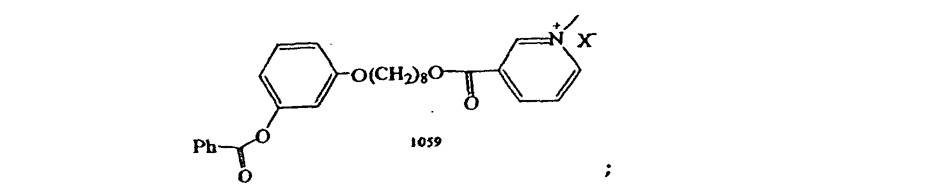 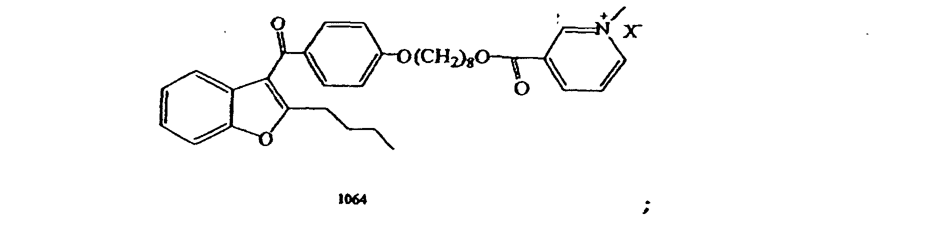 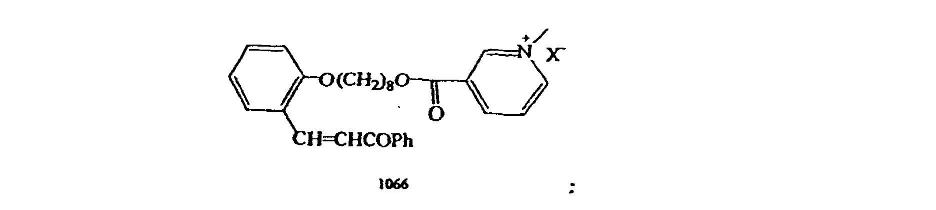 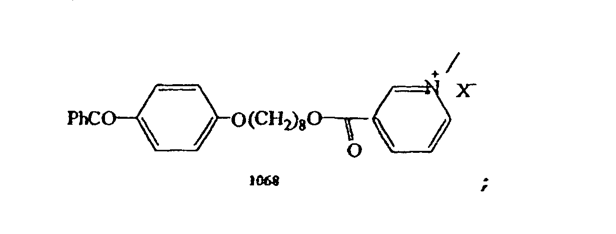 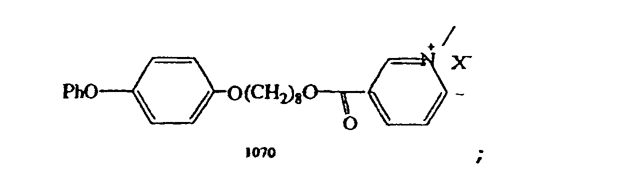 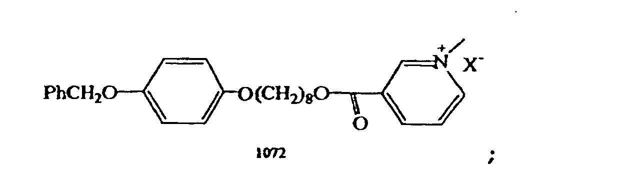 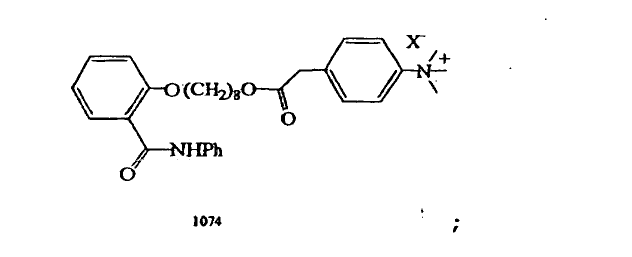 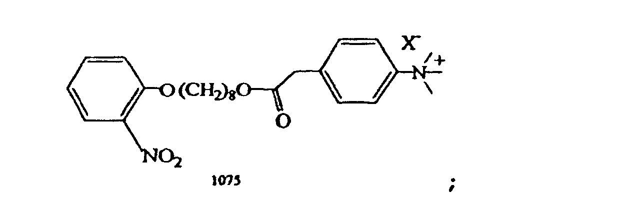 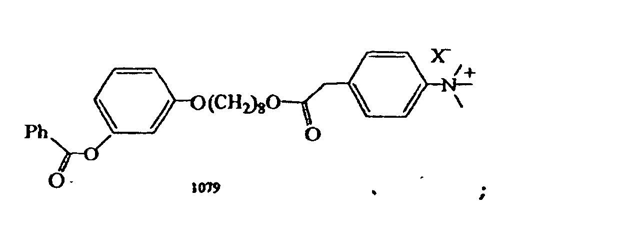 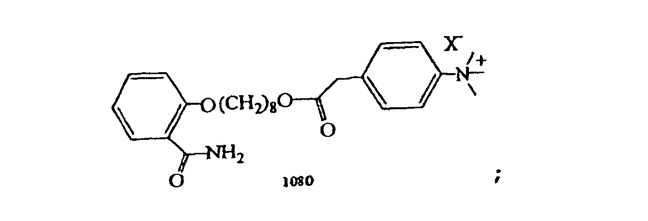 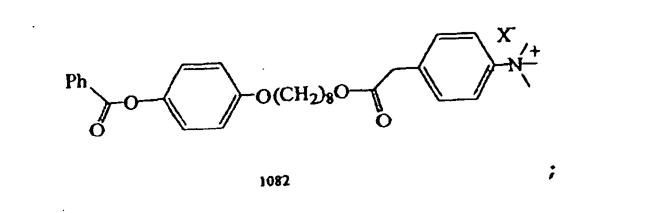 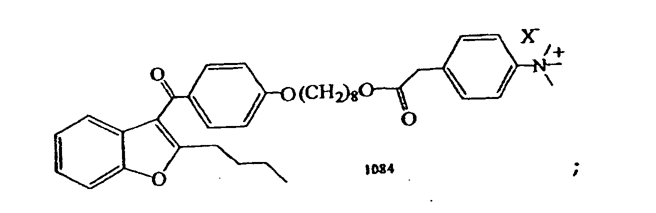 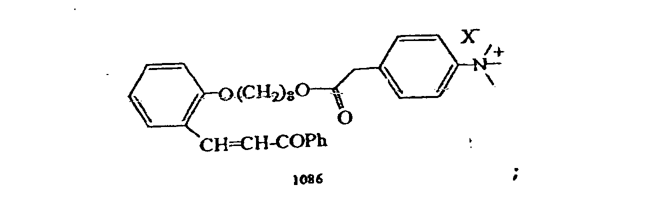 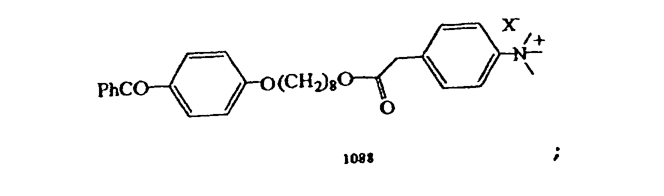 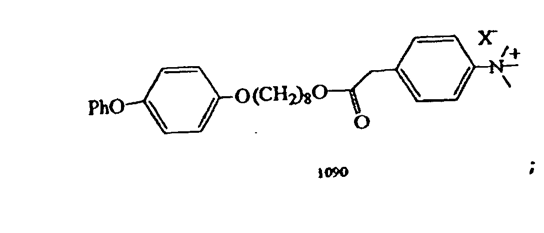 ou

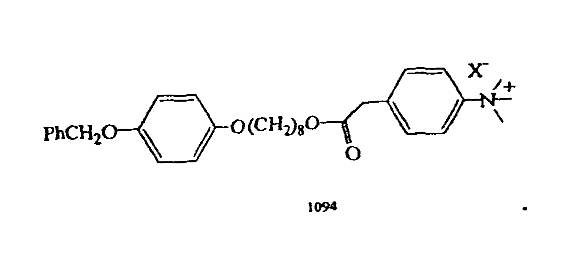

14. Composé selon la revendication 10, qui a la formule

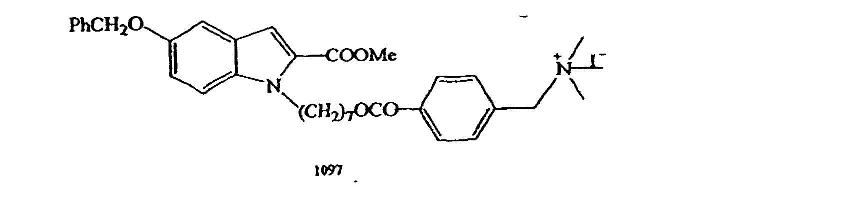

15. Composé de formule : 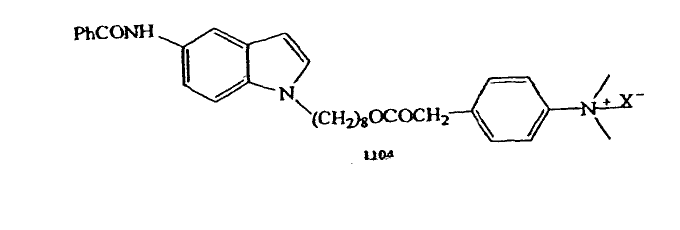 ou 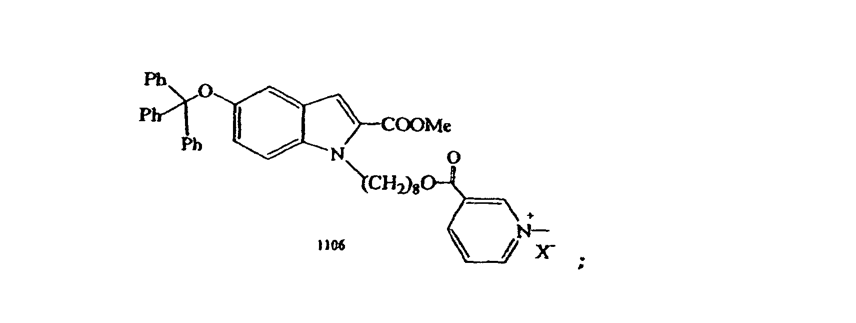 où X⁻ est un anion.

16. Composé selon la revendication 13, qui a la formule :

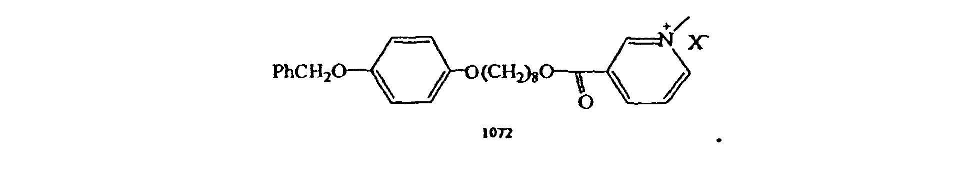

17. Composé selon la revendication 13, qui a la formule :

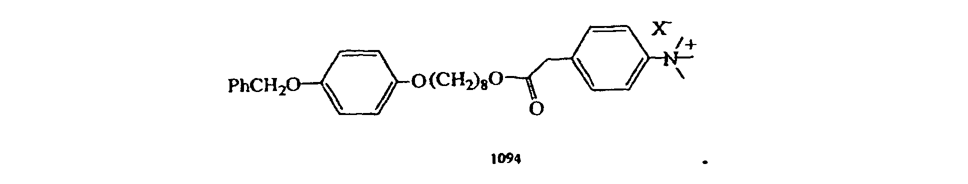

18. Composé selon l'une quelconque des revendications 10 à 17, qui inhibe l'enzyme NAD synthétase d'un microbe.

19. Composé selon l'une quelconque des revendications 1 à 18, qui inhibe la croissance du microbe avec une CI₅₀ d'environ 100 µM ou moins.

20. Composé selon l'une quelconque des revendications 1 à 19, qui se lie à au moins un site récepteur de l'enzyme NAD synthétase d'un microbe.

21. Formulation qui comprend en tant que principe actif un composé selon l'une quelconque des revendications 1 à 20 associé à un ou plusieurs supports, agents médicamenteux, agents pharmaceutiques, adjuvants ou diluants acceptables.

22. Composé selon l'une quelconque des revendications 1 à 20 utile en médecine.

23. Utilisation du composé selon l'une quelconque des revendications 1 à 20 dans la fabrication d'un médicament pour le traitement ou la prévention d'une infection bactérienne.

24. Produit pharmaceutique pour la prévention ou le traitement d'une infection bactérienne, **caractérisé en ce qu'**il contient un composé selon l'une quelconque des revendications 1 à 20 en tant que principe actif.

25. Procédé de préparation d'un composé inhibiteur de NAD synthétase bactérienne comprenant :
(a) la bromation d'une aniline avec du N-bromosuccinimide pour former une 2-bromo-aniline substituée par R¹ ou une 2-bromo-aniline substituée par R² ;
(b) la réaction de la 2-bromo-aniline substituée par R¹ ou de la 2-bromo-aniline substituée par R² selon une réaction de couplage de Heck pour former une aniline alcyne-substituée ;
(c) la réaction de l'aniline alcyne-substituée selon une réaction de cyclisation pour former un indole alcool ;
(d) la quaternisation de l'indole alcool avec une amine ;
(e) la réaction de l'indole alcool avec de l'anhydride trifluorométhylsulfonique pour fournir un triflate d'indole ; et
(f) la réaction du triflate d'indole avec une amine pour former un produit indole alkylammonium.

26. Procédé de préparation d'un composé inhibiteur de NAD synthétase bactérienne comprenant :
(a) l'alkylation d'un phénol avec du 7-bromo-1-heptanol pour fournir le 7-(phényloxy)-1-heptanol ;
(b) la mésylation du 7-(phényloxy)-1-heptanol pour fournir du méthanesulfonate de 7-(phényloxy)-1-heptyle ;
(c) l'estérification du méthanesulfonate de 7-(phényloxy)-1-heptyle pour fournir le nicotinate de 7-(phényloxy)-1-heptyle ; et
(d) la N-méthylation du nicotinate de 7-(phényloxy)-1-heptyle pour fournir l'iodure de [nicotinate de 7-(phényloxy)-1-heptyle-(N-méthyl)].

27. Composition désinfectante comprenant un composé selon l'une quelconque des revendications 1 à 20.
